(19) **Europäisches Patentamt**
**European Patent Office**
**Office européen des brevets**

(11) **EP 2 557 421 A2**

(12) **EUROPEAN PATENT APPLICATION**

(43) Date of publication:
**13.02.2013 Bulletin 2013/07**

(51) Int Cl.:
***G01N 33/50*** (2006.01)    ***G01N 33/68*** (2006.01)

(21) Application number: **12173637.5**

(22) Date of filing: **19.03.2008**

<table>
<tr><td>

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **23.03.2007 GB 0705631**
**05.06.2007 GB 0710775**

(62) Document number(s) of the earlier application(s) in accordance with Art. 76 EPC:
**08718821.5 / 2 142 923**

(71) Applicant: **KING'S COLLEGE LONDON**
**London WC2R 2LS (GB)**

</td><td>

(72) Inventor: **Malik, Afshan**
**London, SE11 9NN (GB)**

(74) Representative: **Holliday, Louise Caroline**
**D Young & Co LLP**
**120 Holborn**
**London EC1N 2DY (GB)**

Remarks:
•This application was filed on 26-06-2012 as a divisional application to the application mentioned under INID code 62.
•Claims filed after the date of receipt of the divisional application (Rule 68(4) EPC.).

</td></tr>
</table>

(54) **ALTERED MITOCHONDRIAL ACTIVITY IN DISEASES RESULTING FROM OXIDATIVE STRESS**

(57)    In one aspect, there is provided a method for identifying a subject having or at risk of developing one or more diseases resulting from oxidative stress comprising the steps of: (a) providing a test sample from said subject; and (b) measuring at least one indicator of altered mitochondrial activity in the test sample, wherein a difference in mitochondrial activity in the test sample as compared to a control is indicative that said subject has or is at risk of developing said diseases.

**Figure 1**

Mitochondrial copy numbers in normal (c) and diabetic (GK) rat kidneys

EP 2 557 421 A2

SEQUENCE LISTING

<110> King's College London

<120> METHOD

<130> P026963WO

<140> PCT/GB2008/000984
<141> 2008-03-19

<150> GB 0705631.0
<151> 2007-03-23

<150> GB 0710775.8
<151> 2007-06-05

<160> 10

<170> PatentIn version 3.5

<210> 1
<211> 16
<212> DNA
<213> Artificial Sequence

<220>
<223> Oligonucleotide primer

<400> 1
ctgccgagac gtaaac                                                    16


<210> 2
<211> 16
<212> DNA
<213> Artificial Sequence

<220>
<223> Oligonucleotide primer

<400> 2
ttatcgccgc ccttgc                                                    16


<210> 3
<211> 19
<212> DNA
<213> Artificial Sequence

<220>
<223> Oligonucleotide primer

<400> 3
tgccattcag aaaactccc                                                 19


<210> 4
<211> 20
<212> DNA
<213> Artificial Sequence

<220>
<223> Oligonucleotide primer

```
<400>   4
acatcctggc tcacactgaa                                          20


<210>   5
<211>   20
<212>   DNA
<213>   Artificial Sequence

<220>
<223>   Oligonucleotide primer

<400>   5
acggtcaggt catcactatc                                          20


<210>   6
<211>   19
<212>   DNA
<213>   Artificial Sequence

<220>
<223>   Oligonucleotide primer

<400>   6
tctgtgtgga ttggtggct                                           19


<210>   7
<211>   35
<212>   DNA
<213>   Artificial Sequence

<220>
<223>   Oligonucleotide primer

<400>   7
cctcatccta gcaataatcc ccatcctcca tatat                         35


<210>   8
<211>   25
<212>   DNA
<213>   Artificial Sequence

<220>
<223>   Oligonucleotide primer

<400>   8
acccttttac catcattgga caagt                                    25


<210>   9
<211>   23
<212>   DNA
<213>   Artificial Sequence

<220>
<223>   Oligonucleotide primer

<400>   9
ctgtctccat gtttgatgta tct                                      23


<210>   10
```

```
<211>   24
<212>   DNA
<213>   Artificial Sequence

<220>
<223>   Oligonucleotide primer

<400>   10
caacttagag gtggggagca gaga                                              24
```

## Description

FIELD

The present invention relates, in one general aspect, to the identification of a subject having or at risk of developing a disease resulting from oxidative stress in the body. The present invention also relates, in another general aspect, to the identification of a diabetic subject having or at risk of developing microvascular disease, kidney disease and/or cardiovascular disease.

BACKGROUND

Oxidative stress results from an imbalance between the generation of reactive oxygen species (ROS) and the body;s anti-oxidative response. In the cell, ROS is produced mainly via the mitochondrial electron transport chain and via the membrane bound NADPH oxidase through electron leakage. The cell is protected from this ROS by both enzymatic (*eg*. catalase, SOD and/or glutothione peroxidise) and non-enzymatic (*eg*. vitamin E, vitamin C and/or uric acid) mechanisms.

Diabetes mellitus, more commonly known as diabetes, is a disease in which the body does not produce and/or properly use insulin, a hormone that aids the body in converting sugars and other foods into energy. In a non-diabetic individual, insulin is produced in the pancreas at the islets of Langerhans in response to an increase of glucose in the gut and/or blood. Insulin then acts in conjunction with the liver to control glucose metabolism in the body. While diabetes is typically thought of as a blood-sugar disease, diabetes may result in numerous life-threatening complications. For example, diabetes may lead to various diseases - such as retinopathy, nephropathy, and neuropathy.

Kidney disease is another a complication of diabetes where the kidney loses its ability to function properly. About one in four people with diabetes might go on to develop kidney disease.

One particular disease that afflicts diabetics is diabetic nephropathy. The pathological hallmark of diabetic nephropathy is glomerulosclerosis due to accumulation of extracellular matrix proteins in the glomerular mesangium. Mesangial matrix accumulation reflects both increased synthesis and decreased degradation of extracellular matrix (ECM) components, and correlates with the clinical onset of proteinuria, hypertension and progressive kidney failure. Hyperglycaemia is a major stimulus for mesangial cell matrix production in diabetic nephropathy. The mechanisms by which hyperglycaemia perturb mesangial cell function are still being appreciated and include direct effects of high extracellular glucose levels and indirect effects transduced through alterations in glomerular haemodynamics and through the actions of advanced glycosylation end products.

Studies have shown that diabetic nephropathy is the leading cause of end-stage renal disease - complete kidney failure necessitating dialysis or transplantation. There are currently around 32,000 people in the UK on renal replacement therapy (RRT). Around 10 per cent (~3,200) of these have diabetes. Every year around 5,500 people begin RRT. Sixteen per cent of these people have diabetes. Around eight per cent of people with diabetes in the UK are receiving some form of treatment for kidney disease. Diabetes currently affects >170 million people worldwide and is predicted to affect twice this number in the next 30 years. A large increase in microvascular complications is likely to follow the global epidemic of diabetes, as 30-50% of diabetics would be predicted to develop nephropathy, and a greater number will develop retinopathy and or neuropathy (Wild S et al. (2004) Diabetes Care 27: 1047-1053).

The earliest detectable change in the course of diabetic nephropathy is that the kidney may start allowing more albumin (plasma protein) than normal in the urine (albuminuria). As diabetic nephropathy progresses the amount of albumin being excreted in the urine increases. For this reason, diabetic nephropathy is currently diagnosed by measuring protein leakage into the urine (micoralbuminuria). However, micoralbuminuria usually only occurs after the disease has become established in the subj ect.

Mitochondrial DNA (mtDNA) is related to the pathogenesis of diabetes (Gerbitz, K. D. et al., Biochim. Biophys. Acta, 1271, 253-260(1995)). Most pathological studies on mtDNA have focused on its biochemical nature, such as mutation or deletion thereof (Shoffner, J. M. and Wallace, D. C., "Oxidative phosphorylation diseases" in The Metabolic and Molecular Bases of Inherited Disease, Vol. 1, pp 1535-1610. Scrier C. R., Beaudet A. L., Sly W. S., Valle D., ded., McGraw Hill, International Edition), while only a limited number of studies have dealt with the quantitative aspect of mtDNA. Shin, C. S., J. Kor. Diabetes Asso., 18, 344-350 (1994) reported that the amount of mtDNA in peripheral blood of a diabetic patient is lower than normal. Moreover, as reviewed by Rolo & Palmeira (2006) Toxicology and Applied Pharmacology 212, 167-178 a decrease in mitochondrial DNA copy number has been linked to the pathogenesis of type 2 diabetes.

There is a need to develop improved diagnostic tests to detect and/or monitor the onset and progression of one or more diseases resulting from oxidative stress in the body. In particular there is a need for the identification of an improved risk factor that can be used to more accurately predict the occurrence and/or identify such disease(s) in patients and so the disease(s) can be treated, inhibited, limited or prevented. In particular, in view of the high morbidity and mortality rate from diseases in diabetic subjects - such as diabetic nephropathy - there is a need to develop improved diagnostic tests to detect and/or monitor the onset and progression of such diseases. In particular there is a need for the identification of an improved risk factor that can be used to more accurately predict the occurrence and/or identify disease in diabetic patients and so the disease can be treated, inhibited, limited or prevented.

SUMMARY OF THE INVENTION

The present invention broadly relates to the identification of a risk factor associated with disease resulting from oxidative stress in a subject - such as a diabetic subject.

Without wishing to be bound by any particular theory it is believed that the imbalance that leads to oxidative stress could be caused by altered mitochondrial activity - such as an altered mitochondrial genome relative to nuclear genome ratio, as described herein. A chronic increase in reactive oxygen species from altered (eg. elevated) mitochondrial activity may result in the cells normal anti-oxidative response to be overloaded resulting in elevated reactive oxygen species causing damage to for example, membranes, lipids, DNA and proteins which may then lead to disease. Free radicals/reactive oxygen species have been linked in the last few decades to many diseases (see for example, Flora (2007) Role of free radicals in antioxidants in health and disease Cell Mol. Biol, 53-1:1-2). Therefore, diseases which are a result of or linked to oxidative stress in the body, may be candidates for testing using the methods described herein.

The present invention is also based, at least in part, on the surprising finding that altered (eg. increased or enhanced) mitochondrial activity in a diabetic subject is indicative that they are at risk of or have developed a disease resulting from oxidative stress - such as a microvascular disease, kidney disease and/or cardiovascular disease. In diabetic subjects that have or are at risk of developing such disease, the number of mitochondria is increased. Surprisingly, this is opposite to the pathogenesis of type 2 diabetes in which a decrease in mitochondrial DNA copy number has been observed.

The earlier a diabetic patient who is developing or likely to develop a disease as described herein can be identified the more effective the preventative treatments available are. Accordingly, the present invention is particularly advantageous since diabetic subjects that are at risk of developing disease as described herein may be identified. Moreover, patients that have already developed disease may be identified at an earlier stage. Advantageously, the methodology described herein may be used at regular diabetic clinics to identify patients who are likely to develop or have disease at an earlier stage than using the diagnostic tests of the prior art. Advantageously, the methodology described herein could be used on a regular basis to patients. It could even be offered to patients at risk of diabetes i. e. prediabetics, and to all newly diagnosed diabetics.

One important goal is to be able to identify patients who are likely to develop disease as described herein before organ damage occurs. The identified patients may be offered lifestyle advice, palliative care and/or treatment to prevent the onset of the disease, or to slow or regress the progression of the disease.

SUMMARY ASPECTS

In one aspect, there is provided a method for identifying a subject having or at risk of developing one or more diseases resulting from oxidative stress comprising the steps of: (a) providing a test sample from said subject; and (b) measuring at least one indicator of altered mitochondrial activity in the test sample, wherein a difference in mitochondrial activity in the test sample as compared to a control is indicative that said subject has or is at risk of developing said diseases.

In a further aspect, there is provided a method for measuring mitochondrial copy number in a cell comprising the steps of: (a) amplifying a single copy nuclear gene; (b) amplifying a mitochondrial gene; (c) quantifying the amount of amplification product from step (a) and step (b); and (d) calculating the mitochondrial copy number in the cell.

In a further aspect, there is provided an assay method for identifying one or more agents that modulate one or more diseases resulting from oxidative stress in a subject, comprising the steps of: (a) providing a sample comprising one or more mitochondria; (b) contacting said sample with one or more agents; and (c) measuring the activity of the mitochondria in the sample in the presence and absence of said agent(s), wherein a difference between a) the mitochondrial activity in the presence of the agent and b) the mitochondrial activity in the absence of the agent is indicative that the agent(s) can modulate the one or more disease(s) in the subject.

In another aspect, there is provided an agent obtained or obtainable by the assay method

There is also provided a method for preventing and/or treating one or more diseases resulting from oxidative stress in a subject comprising administering to said subject one or more agents described herein and/or one or more agents that modulate mitochondrial activity.

Another aspect relates to an agent for use in the prevention and/or treatment of one or more diseases resulting from oxidative stress.

Another aspect relates to an agent for the prevention and/or treatment of one or more diseases resulting from oxidative stress.

Another aspect relates to the use of an agent described herein and/or an agent that modulates mitochondrial activity in the manufacture of a medicament for the prevention and/or treatment of one or more diseases resulting from oxidative stress.

In one further aspect, there is also provided a method for identifying one or more genes that are involved in the presentation of one or more diseases resulting from oxidative stress, comprising the step of: (a) providing a test sample from a subject suffering from one or more diseases resulting from oxidative stress; and (b) determining the expression profile of one or more mitochondrial genes in said test sample, wherein a difference in the expression profile of one or more mitochondrial genes in said test sample as compared to a sample from a control subject is indicative that said gene(s) are involved in the presentation of said disease(s) in a subject.

A gene(s) obtained or obtainable by this method is also provided.

There is also provided a method of determining oxidative stress in a sample, comprising the step of: measuring at least one indicator of altered mitochondrial activity in the test sample, wherein a difference in mitochondrial activity in the test sample as compared to a control is indicative of oxidative stress. This method may provide a measure of overall oxidative stress.

Finally, there is provided a method, an assay method, an agent, a use or a gene substantially as described herein with reference to the accompanying Figures.

SUMMARY EMBODIMENTS

In one embodiment, the subject is a diabetic subject.

In one embodiment, the disease is selected from the group consisting of microvascular disease, kidney disease, cardiovascular disease, schizophrenia, Parkinsons disease, Huntingdon's chorea, metal-induced toxicity in diabetes, Alzheimers disease, cancer, anaemia, hypertension, atherosclerosis, diabetes, muscular dystrophy and aging or a combination of at least two of said diseases.

In another embodiment, the difference in mitochondrial activity is an increase in mitochondrial activity.

In another embodiment, the at least one indicator of mitochondrial activity is the number of mitochondria in the test sample as compared to the control.

In another embodiment, the number of mitochondria in the test sample as compared to the control is determined using oxygen uptake measurements, measuring cytochrome oxidiase activity and/or counting the number of mitochondria using a microscope.

In another embodiment, the at least one indicator of mitochondrial activity is the copy number of the mitochondrial genome in the test sample as compared to the control.

In another embodiment, the copy number of the mitochondrial genome is determined by comparing it to the copy number of the nuclear genome.

In another embodiment, the copy number is determined using PCR amplification.

In another embodiment, the copy number is determined using real-time PCR amplification.

In another embodiment, the test sample is or is derived from a blood sample, a biopsy specimen, a tissue explant, or an organ culture.

In another embodiment, the test sample is nucleic acid that is derived from a blood sample, a biopsy specimen, a tissue explant, or an organ culture.

In another embodiment, the nucleic acid is genomic DNA and/or total RNA.

In another embodiment, the test sample is a cultured cell.

In another embodiment, the cultured cell is a cultured renal cell.

In another embodiment, the renal cell is a mesangial cell or a tubular cell.

In another embodiment, the cultured cell is grown in high or low glucose prior to measuring the at least one indicator of mitochondrial activity.

In another embodiment, the microvascular disease is diabetic nephropathy, diabetic retinopathy and/or diabetic neuropathy.

In another embodiment, the kidney disease is kidney disease associated with hypertension, kidney disease associated with metabolic syndrome, kidney disease associated with gloemrulonephritis and/or cardiovascular disease.

In another embodiment, the single copy region of the nuclear genome is a single copy nuclear gene.

In another embodiment, the single copy nuclear gene is actin or β2microglobulin.

In another embodiment, the region of the mitochondrial genome is a mitochondrial gene.

In another embodiment, the mitochondrial gene is cytochrome B.

In another embodiment, said agent(s) modulate mitochondrial activity in a renal cell.

Further aspects of the invention are set out below in the following numbered paragraphs:-

1. A method for identifying a subject having or at risk of developing one or more diseases resulting from oxidative stress comprising the steps of:

(a) providing a test sample from said subject; and

(b) measuring at least one indicator of altered mitochondrial activity in the test sample,

wherein a difference in mitochondrial activity in the test sample as compared to a control is indicative that said subject has or is at risk of developing said disease(s).

2. A method according to paragraph 1, wherein the subject is a diabetic subject.

3. A method according to paragraph 1 or paragraph 2, wherein the disease is selected from the group consisting of microvascular disease, kidney disease, cardiovascular disease, schizophrenia, Parkinsons disease, Huntingdon's chorea, metal-induced toxicity in diabetes, Alzheimers disease, cancer, anaemia, hypertension, atherosclerosis, diabetes, muscular dystrophy and aging or a combination of at least two of said diseases.

4. The method according to any of paragraphs 1 to 3, wherein the difference in mitochondrial activity is an increase in mitochondrial activity.

5. The method according to any of paragraphs 1 to 4, wherein the at least one indicator of altered mitochondrial activity is the number of mitochondria in the test sample as compared to the control.

6. The method according to paragraph 5, wherein the number of mitochondria is measured using oxygen uptake measurements, measuring cytochrome oxidiase activity and/or counting the number of mitochondria using a microscope.

7. The method according to any of paragraphs 1 to 4, wherein the at least one indicator of altered mitochondrial activity is the copy number of the mitochondrial genome in the test sample as compared to the control.

8. The method according to paragraph 7, wherein the copy number of the mitochondrial genome is determined by comparing it to the copy number of the nuclear genome.

9. The method according to paragraph 7 or paragraph 8, wherein the copy number is determined using PCR amplification.

10. The method according to any of paragraphs 7 to 9, wherein the copy number is determined using real-time PCR amplification.

11. The method according to any of the preceding paragraphs, wherein the test sample is or is derived from a blood sample, a biopsy specimen, a tissue explant, an organ culture or a cultured cell.

12. The method according to any of the preceding paragraphs, wherein the test sample is nucleic acid that is or is derived from a blood sample, a biopsy specimen, a tissue explant, an organ culture or a cultured cell.

13. The method according to paragraph 12, wherein the nucleic acid is genomic DNA and/or total RNA.

14. The method according to any of paragraphs 11 to 13, wherein the cultured cell is a cultured renal cell.

15. The method according to the paragraph 14, wherein the cultured renal cell is a mesangial cell or a tubular cell.

16. The method according to any of paragraphs 12 to 15, wherein said cultured cell is grown in high or low glucose prior to measuring the at least one indicator of mitochondrial activity.

17. The method according to any of paragraphs 3 to 16, wherein the microvascular disease is diabetic nephropathy, diabetic retinopathy and/or diabetic neuropathy.

18. The method according to any of paragraphs 3 to 16, wherein the kidney disease is kidney disease associated with hypertension, kidney disease associated with metabolic syndrome, kidney disease associated with gloemrulonephritis and/or cardiovascular disease.

19. A method for measuring mitochondrial copy number in a cell comprising the steps of:

(a) amplifying a single copy region of the nuclear genome;

(b) amplifying a region of the mitochondrial genome;

(c) quantifying the amount of amplification product from step (a) and step (b); and

(d) calculating the mitochondrial copy number in the cell.

20. The method according to paragraph 19, wherein the single copy region of the nuclear genome is a single copy nuclear gene.

21. The method according to paragraph 19 or paragraph 20, wherein the single copy nuclear gene is actin or β2microglobulin.

22. The method according to any of paragraphs 19 to 21, wherein the region of the mitochondrial genome is a mitochondrial gene.

23. The method according to paragraph 22, wherein the mitochondrial gene is cytochrome B.

24. An assay method for identifying one or more agents that modulate one or more diseases resulting from oxidative stress in a subject, comprising the steps of:

(a) providing a sample comprising one or more mitochondria;

(b) contacting said sample with one or more agents; and

(c) measuring the activity of the mitochondria in the sample in the presence and absence of said agent(s),

wherein a difference between a) the mitochondrial activity in the presence of the agent and b) the mitochondrial activity in the absence of the agent is indicative that the agent(s) can modulate the one or more disease(s) in the subject.

25. The method according to paragraph 24, wherein the disease is is selected from the group consisting of microvascular disease, kidney disease, cardiovascular disease, schizophrenia, Parkinsons disease, Huntingdon's chorea, metal-induced toxicity in diabetes, Alzheimers disease, cancer, anaemia, hypertension, atherosclerosis, diabetes, muscular dystrophy and aging or a combination of at least two of said diseases.

26. The method according to paragraph 24 or paragraph 25, wherein the subject is a diabetic subject.

27. An agent obtained or obtainable by the method of any of paragraphs 24 to 26.

28. A method for preventing and/or treating one or more diseases resulting from oxidative stress in a subject comprising administering to said subject one or more agents according to paragraph 27 and/or one or more agents that modulate mitochondrial activity.

29. The method according to paragraph 28, wherein the disease is selected from the group consisting of microvascular disease, kidney disease, cardiovascular disease, schizophrenia, Parkinson's disease, Huntingdon's chorea, metal-induced toxicity in diabetes, Alzheimer's disease, cancer, anemia, hypertension, atherosclerosis, diabetes, muscular dystrophy and aging or a combination of at least two of said diseases.

30. The method according to paragraph 28 or paragraph 29, wherein the subject is a diabetic subject.

31. An agent according to paragraph 27 for use in the prevention and/or treatment of one or more diseases resulting



**EP 2 557 421 A2**

from oxidative stress.

32. An agent according to paragraph 27 for the prevention and/or treatment of one or more diseases resulting from oxidative stress.

33. Use of an agent according to paragraph 27 and/or agent that modulates mitochondrial activity in the manufacture of a medicament for the prevention and/or treatment of one or more diseases resulting from oxidative stress.

34. The agent according to paragraph 31 or paragraph 32 or the use according to claim 33, wherein said disease is selected from the group consisting of microvascular disease, kidney disease, cardiovascular disease, schizophrenia, Parkinsons disease, Huntingdon's chorea, metal-induced toxicity in diabetes, Alzheimers disease, cancer, anaemia, hypertension, atherosclerosis, diabetes, muscular dystrophy and aging or a combination of at least two of said diseases.

35. The method according to any of paragraphs 28 to 30, the agent according to paragraph 31 or paragraph 32 or the use according to paragraph 33 or paragraph 34, wherein said agent(s) modulate mitochondrial activity in a renal cell.

36. A method for identifying one or more genes that are involved in the presentation of one or more diseases resulting from oxidative stress, comprising the step of:

(a) providing a test sample from a subject suffering from one or more diseases resulting from oxidative stress; and

(b) determining the expression profile of one or more mitochondrial genes in said test sample,

wherein a difference in the expression profile of one or more mitochondrial genes in said test sample as compared to a sample from a control subject is indicative that said gene(s) are involved in the presentation of said disease(s) in a subject.

37. The method according to paragraph 36, wherein the disease is selected from the group consisting of microvascular disease, kidney disease, cardiovascular disease, schizophrenia, Parkinsons disease, Huntingdon's chorea, metal-induced toxicity in diabetes, Alzheimers disease, cancer, anaemia, hypertension, atherosclerosis, diabetes, muscular dystrophy and aging or a combination of at least two of said diseases.

38. The method according to paragraph 36 or paragraph 37, wherein the subject is a diabetic subject.

39. A gene(s) obtained or obtainable by the method according to any of paragraphs 36 to 38.

40. A method of determining oxidative stress in a sample, comprising the step of: measuring at least one indicator of altered mitochondrial activity in the test sample, wherein a difference in mitochondrial activity in the test sample as compared to a control is indicative of oxidative stress.

41. A method, an assay method, an agent, a use or a gene substantially as described herein with reference to the accompanying Figures.

<u>FIGURES</u>

*Figure 1*
Genomic DNA was isolated from kidneys of control (normoglycemic Wistars) and diabetic (hyperglycemic GK) rats. Real time qPCR was used to measure the copy numbers of rCYTB (mitochondrially encoded) and rACtin (nuclear encoded). Two different sets of primers were used to measure rCYTB, each qPCR was carried out in duplicate and the results from both primers were averaged. The ratio of rCYTB/Actin represents the Mt/N ratio.

*Figure 2*
Human mesangial cells were cultured in triplicate in either 5mM glucose (NG) or 25mM glucose (HG) for 4 days, agter which the genomic DNA was extracted and the ratio of hCYTB to hActin was determined using real time qPCR.

The data shown are averages of 3 experiments.

*Figure 3*

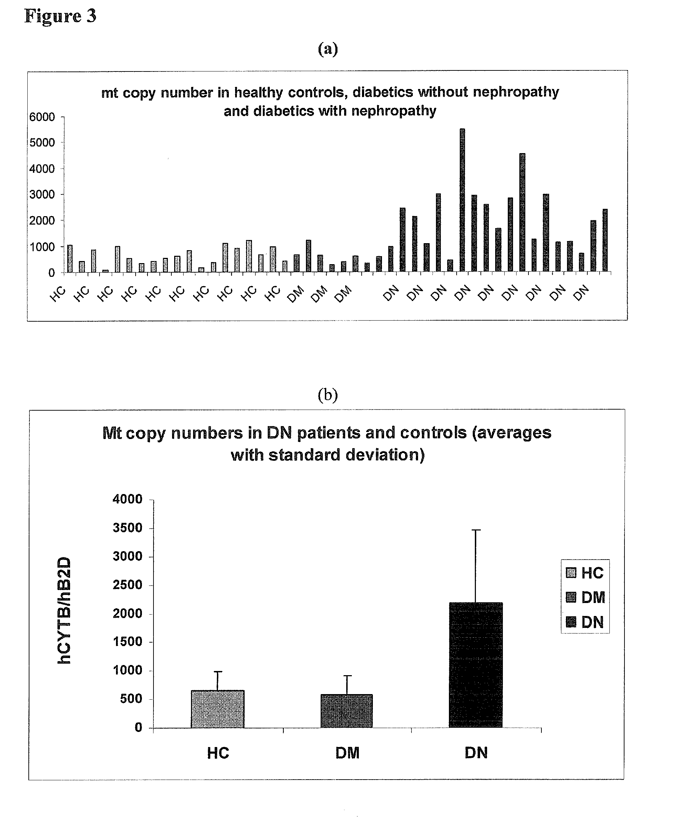

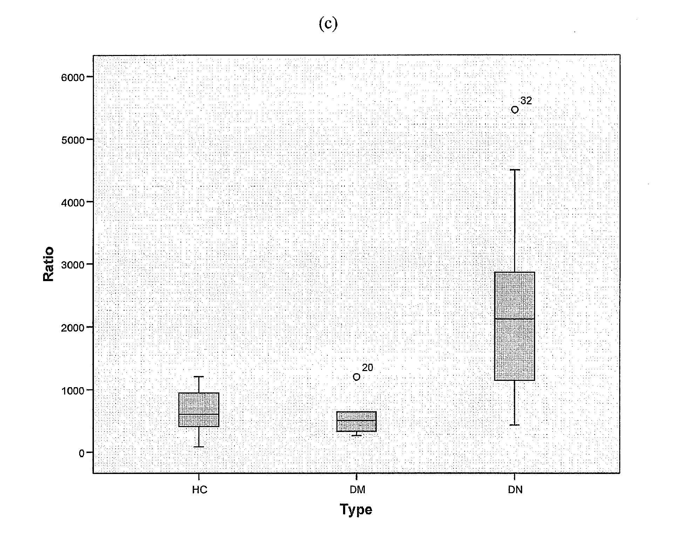

Mitochondrial copy numbers were determined in healthy controls (HC), diabetics without nephropathy (DM) and diabetic nephropaths (DN) by quantitating hCYTB and hB2M. Figure 3a shows individual patient results; Figure 3b shows averaged results for the 3 groups; Figure 3c is a boxplot showing the range, the mean values and overlap between the groups. The single values for patients 20 and 32 fall outside the range.

DETAILED DESCRIPTION

OXIDATIVE STRESS

As described above, oxidative stress results from an imbalance between the generation of reactive oxygen species (ROS) and the body's anti-oxidative response.

The manifestation of oxidative stress in the blood plasma can be described by two detectable components namely the decrease in the plasma concentration of acid-soluble thiol (essentially reduced cysteine) and by the increase of the plasma concentration of cysteine disulfide (cysteine). The ratio of these two components is an indicator of the thiol/disulfide redox state and is a manifestation of oxidative stress that can be tested (Gohil et al., L Appl. Physiol. 64 (1988) 115-119; Duthie et al., Arch. Biochem. Biophys. 282 (1990) 78-83; Sastre et al., Am. J. Physiol. 263 (1992) R992-R995; Sen et al., J. Appl. Physiol. 76 (1994) 2570-2577; Hack et al., BLOOD 92 (1998) 59-67). Further methods for measuring oxidative stress are also well known in the art, in for example, US 6,852,541, US 6,541,267 and US 6,096,556.

Non-limiting examples of diseases (*eg.* aging) linked to oxidative stress are described below.

*Schizophrenia, Parkinsons disease & Huntingdon's chorea*

There are numerous models proposed to explain the occurrence of schizophrenia Parkinsons disease & Huntingdon's chorea which converge on neuronal membranes which contain a high proportion of polyunsaturated fatty acids and is the site for oxidative stress. There is accumulating evidence for oxidative membrane damage (Fendri et al., Oxidative stress: involvement in schizophrenia pathophysiology:a review Encephale: 2006 Mar-Apr;32 (2Pt1):244-52) which may be caused by an increased mitochondrial genome relative to nuclear genome ratio.

*Metal-induced toxicity in Diabetes, Alzheimers type dementia, Cancer, other metal related diseases e.g. anaemia.*

There is much evidence in the literature showing the neuropotective and neurodegenerative role of zinc and copper in the aetiology of Alzheimer's disease. Similarly a link between vanadium, oxidative stress and diabetes is also shown. The unifying factor in determining the toxicity and carcinogenesis of metals is the generation of reactive oxygen species (Valko et al., (2005) Metals, toxicity and oxidative stress. Current Medicinal Chemistry, 12 (10):1161-1208). This may be connected to the mitochondrial activity since mitochondria are the major site of reactive oxygen species production.

*Oxidative stress and Cancer*

The process of carcinogenesis consists of numerous distinct stages, each with different underlying mechanisms. Reactive oxygen species play an initiator role through causing DNA damage which can then lead to apoptosis (signalled through the mitochondria) or proliferation, both of which take place via complex signalling molecules. The list of signalling molecules involved in carcinogenesis is large and many of these are directly affected by altered reactive oxygen species levels. (Valko et al., 2006, Free radicals, metals and antioxidants in oxidative stress-induced cancer Chemico-biological Interactions 160:1-40).

*Cardiovascular disease, Hypertension, Atherosclerosis, Diabetes*

The involvement of oxidative stress in these diseases is described in, for example, Willcox et al., (2004) Antioxidants and prevention of chronic disease. Crtic Rev. Food Sci. Nutr. 44: 275-295; and B.Van Houten et al. (2006) Role of mitochondrial DNA in toxic responses to oxidative Stress. DNA Repair (Amst) 5:145-152.

*Muscular dystrophy*

The involvement of oxidative stress in muscular dystrophy is described in Tidball and Wehlin-Henricks (2007) The role of free radicals in the pathophysiology of muscular dystrophy. J Appl Physiol. 102:1677-1686.

DIABETES

Several types of diabetes exist. Insulin dependent diabetes mellitus (IDDM), commonly referred to as Type 1 diabetes, is an auto-immune disease that affects the islets of Langerhans, destroying the body's ability to produce insulin. Non-insulin dependent diabetes mellitus (NIDDM), commonly referred to as Type 2 diabetes, is a metabolic disorder resulting from the body's inability to produce enough insulin or properly use the insulin produced. Type 2 diabetes is usually associated with obesity and a sedentary lifestyle.

The term "diabetic" includes, but is not limited to, individuals with Type 1 diabetes, Type 2 diabetes, gestational diabetes, and any other conditions of insulin deficiency.

In one embodiment, the diabetic subject is a subject suffering from Type 1 diabetes.

In another embodiment, the diabetic subject is a subject suffering from Type 2 diabetes.

DISEASE

In one embodiment, the disease is microvascular disease, kidney disease and/or cardiovascular disease.

In another embodiment, the disease is a disease resulting from oxidative stress in a subject - such as Schizophrenia, Parkinsons disease, Huntingdon's chorea, metal-induced toxicity in diabetes, Alzheimers type dementia, Cancer and other metal related diseases( e.g. anaemia), Cancer, Cardiovascular disease, Hypertension, Atherosclerosis, Diabetes, Muscular dystrophy and/or Aging.

In another embodiment, the disease is a disease associated with diabetes - such as microvascular disease, kidney disease and/or cardiovascular disease.

Microvascular complications of diabetes encompass long term complications of diabetes affecting small blood vessels. These microvascular diseases include retinopathy, nephropathy and neuropathy.

*Retinopathy*

Retinopathy is divided into two main categories. Non-proliferative retinopathy and proliferative retinopathy. Non-proliferative retinopathy can be recognized by development of microaneurysms, venous loops, retinal hemorrhages, hard exudates and soft exudates. Proliferative retinopathy is defined as presence of new blood vessels with or without vitreous hemorrhage. Proliferative retinopathy represents a progression of non-proliferative retinopathy. Microaneurysm formation is the earliest manifestation of diabetic retinopathy. Microaneurysms may form due to release of vasoproliferative factors, weakness in the capillary wall or increased intra-luminal pressures. Microaneurysms can lead to vascular permeability. Vascular permeability in the macula can lead to macular edema and threatens central vision. Obliteration of retinal capillaries can lead to intraretinal microvascular abnormalities (IRMA). As capillary closure becomes extensive, intraretinal hemorrhages develop. Proliferative retinopathy develops due to ischemia and release of vasoactive substances which stimulate new blood vessel formation as a progression of non-proliferative retinopathy. These vessels erupt through the surface of the retina and grow on the posterior surface of the vitreous. These vessels are very friable and can lead to vitreous hemorrhages. The vitreous can contract and lead to retinal detachment.

*Nephropathy*

Diabetic nephropathy is typically described as the presence of persistent proteinuria >0.5 gms/24 hours. Overt nephropathy is characterized by progressive decline in renal function resulting in end stage renal disease. Diabetic nephropathy results from increased glomerular capillary flow which results in increased extracellular matrix production and endothelial damage. This leads to increased glomerular permeability to macromolecules. Mesangial expansion and interstitial sclerosis ensues leading to glomerular sclerosis.

*Neuropathy*

Neuropathy is a heterogenious condition that is associated with nerve pathology. The condition is classified according to the nerves affected. The classification of neuropathy includes focal, diffuse, sensory, motor and autonomic neuropathy. The pathophysiology of neuropathy is complex. Diabetes is associated with dyslipidemia, hyperglycemia, low insulin and growth factor abnormalities. These abnormalities are associated with glycation of blood vessels and nerves. In addition, autoimmunity may affect nerve structure. Trauma and neuroentrapment can lead to structural nerve damage including segmental demyelination, axonal atrophy and loss and progressive demyelination. These effects cause neuropathy. Several agents including laminin B2, IGFI&II, NGF, insulin and NT3 are potential growth factors that may restore nerve function.

Suitably, the microvascular disease is a diabetic microvascular disease - such as diabetic nephropathy, diabetic retinopathy and/or diabetic neuropathy.

Suitably, the disease is another complication of diabetes - such as kidney disease and/or cardiovascular disease.

Specific non-limiting examples of kidney disease include kidney disease associated with hypertension, kidney disease associated with metabolic syndrome and kidney disease associated with gloemrulonephritis.

In one embodiment, the disease is diabetic nephropathy.

DIABETIC NEPHROPATHY

Progression of diabetic nephropathy is characterized by a fairly predictable pattern of events. Generally, the time course of development of diabetic nephropathy is as follows. Glomerular hyperfiltration and renal hypertrophy occur in the first years after the onset of diabetes and are reflected by increased glomerular filtration rate (e.g., from a normal glomerular filtration rate of about 120 ml/min to about 150 ml/min in humans). During the first 5 years of diabetes, pathological changes, such as glomerular hypertrophy, thickening of the glomerular basement membrane, and glomerular mesangial volume expansion, are observed. Glomerular filtration rate gradually returns to normal. After 5 to 10 years of diabetes, individuals begin to excrete small amounts of albumin in the urine (microalbuminuria). Microalbuminuria (diabetic individuals with microalbuminuria are referred to as having incipient diabetic nephropathy) is an important predictor of progression to overt diabetic nephropathy (characterized, in part, by macroalbuminuria or overt proteinuria). The basement membrane thickening and glomerular volume expansion seen in early stages of the disease can accumulate in late stage diabetic nephropathy, leading to obliteration of the capillary lumen, and, eventually, to glomerulosclerosis. Once overt diabetic nephropathy is present, a steady decline in the glomerular filtration rate occurs, and approximately half of individuals reach end-stage renal disease in 7 to 10 years.

Clinically, the stages of development and progression of diabetic nephropathy in humans have been well described. Stage I diabetic nephropathy is associated with increased kidney (i.e., glormerular) filtration (i.e., hyperfiltration, resulting from increased blood flow through the kidneys and glomeruli), increased glomerular filtration rate, glomerular hypertrophy, and enlarged kidneys. Stage II diabetic nephropathy is a clinically silent phase associated with continued hyperfiltration and kidney hypertrophy. Thickening of the glomerular basement membrane and mesangial expansion occurs. Stage III diabetic nephropathy (also known as incipient diabetic nephropathy) is associated with microalbuminuria and micro proteinuria. Microalbuminuria is defined as 30 to 300 mg/day urinary albumin in a 24-hour collection, 20-200 .mu.g/min urinary albumin, or 30 to 300 .mu.g/mg creatinine in a spot collection. The kidneys progressively lose the ability to filter waste and blood levels of creatinine and urea-nitrogen increase. Glomerular basement membrane thickening and mesangial expansion continue to occur with increasing severity. Stage IV diabetic nephropathy (also known as overt diabetic nephropathy) is associated with macroalbuminuria (i.e., clinical albuminuria) and creatinine and blood urea-nitrogen (BUN) levels in the blood continue to rise. Macroalbuminuria is defined as greater than 300 mg/day urinary albumin in a 24-hour collection, greater than 200 μg/min urinary albumin, or greater than 300 μg/mg creatinine spot collection. Once overt diabetic nephropathy occurs, glomerular filtration rate gradually falls over a period of several years. Stage V diabetic nephropathy occurs with end-stage renal disease and kidney failure.

Advantageously, as described herein a risk factor associated with disease - such as diabetic nephropathy - has been identified. The methods of the present invention may be used together with other methods/markers for other risk factors - such as microalbuminuria (a risk factor for diabetic nephropathy and cardiovascular disease) and/or C-reactive protein (a risk factor for cardiovascular disease). These other risk factors are described in Stuveling et al., (2005) Nephrol.Dial.Transpl. 20:497-508

MITOCHONDRIA

Functional mitochondria contain gene products encoded by mitochondrial genes situated in mitochondrial DNA (mtDNA) and by extramitochondrial genes (e.g., nuclear genes) not situated in the circular mitochondrial genome. The 16.5 kb mtDNA encodes 22 tRNAs, two ribosomal RNAs (rRNA) and 13 enzymes of the electron transport chain (ETC), the elaborate multi-complex mitochondrial assembly where, for example, respiratory oxidative phosphorylation takes place.

MITOCHONDRIAL ACTIVITY

As described herein, at least one indicator of altered mitochondrial activity may be used as a marker for one or more of the diseases described herein. Accordingly, a difference (*eg.* an increase or a decrease) in the activity of mitochondria in the test sample as compared to a control is indicative of the presence of, an increased risk of, or a decreased risk of a subject (*eg.* a diabetic subject) developing disease.

The mitochondrial activity in the test sample as compared to a control may be monitored as a preventative measure.

In one embodiment, "altered mitochondrial activity" refers to any structure or activity that is directly or indirectly related to a mitochondrial function that has been changed in a statistically significant manner relative to a control or standard. Altered mitochondrial activity may have its origin in extramitochondrial structures or events as well as in mitochondrial structures or events, in direct interactions between mitochondrial and extramitochondrial genes and/or their gene products, or in structural or functional changes that occur as the result of interactions between intermediates that may be formed as the result of such interactions, including metabolites, catabolites, substrates, precursors, cofactors and the like.

The activity of mitochondria can be modulated by the requirements of a cell and regulated at different levels, for example, by altering copy number, by altering transcriptional levels, and by post transcriptional and post translational modifications. Mitochondrial gene expression may even be regulated by the energy demands of the cell (Mehrabian et al., 2005 J. Neurochemistry, 93:850-860) and it has been shown that cells can respond rapidly to their requirements by altering the rate of transcription. Without being bound by any particular theory, it is believed that the numbers of mitochondrial genomes provide a major regulatory point in activity.

*Number of mitochondria*

In one embodiment, the at least one indicator of altered mitochondrial activity is the number of mitochondria wherein a difference in the number of mitochondria as compared to a control may be indicative of one or more of the diseases described herein.

In another embodiment, the at least one indicator of altered mitochondrial activity is an increase in the number of mitochondria wherein a change (eg. an increase) in the number of mitochondria as compared to a control is indicative of disease.

Methods for determining the number of mitochondria may include, for example, quantitative staining. Typically, quantitative staining of mitochondria may be performed using organelle-selective probes or dyes, including but not limited to mitochondria selective reagents - such as fluorescent dyes that bind to mitochondrial molecular components or potentiometric dyes that accumulate in mitochondria as a function of mitochondrial inner membrane electrochemical potential. Thus, by way of example, mitochondrial number may be quantified by morphometric analysis (e.g., Cruz-Orive et al., 1990 Am. J. Physiol. 258:L148; Schwerzmann et al., 1986 J. Cell Biol. 102:97).

Another method of quantifying mitochondria is to use mass spectrophotomtery (e.g. MSMS) and measuring a specific mitochondrial protein - such as CYTB. Using such methods, blood or urine samples may be used and the system would be high throughput and cost effective. Accordingly, in a further aspect, there is provided a method of quantifying mitochondria comprising the use of mass spectrophotomtery - such as MSMS.

*DNA content of mitochondria*

In another embodiment, the at least one indicator of mitochondrial activity is the mitochondrial DNA content, wherein altered (*e.g.* increased) mitochondrial DNA content as compared to a control may be indicative of disease. Suitably, the mitochondrial DNA content is expressed as a ratio of nuclear genome content.

Quantification of mitochondrial DNA content may be accomplished by various methods that are known in the art - such as oligonucleotide probe hybridization or amplification of nucleic acid using oligonucleotide primers specific for one or more mitochondrial DNA sequences (see, e.g., Miller et al., 1996 J. Neurochem. 67:1897; Fahy et al., 1997 Nucl. Ac. Res. 25:3102; Lee et al., 1998 Diabetes Res. Clin. Practice 42:161; Lee et al., 1997 Diabetes 46(suppl. 1):175A).

The fact that increased mitochondrial genome numbers are indicative of increased mitochondrial activity is evidenced in the literature in, for example, Williams (1986) J. Biol.Chem 261(26):12390-12394), where it is taught that the expression of mitochondrial genes is proportionate to their copy numbers.

In one embodiment, mitochondrial genome number may be measured using an assay based on PCR - such as real time PCR - to measure exact mitochondrial genome copy numbers. In a normal non-dividing cell, there is a single nuclear genome whereas there are hundreds to thousands of copies of the mitochondrial genome. Primers to a single copy nuclear gene and a single mitochondrial gene are designed - such as actin or B2 microglobulin (nuclear gene) and cytochrome B (mitochondrial gene). The PCR products are electrophoresed against a known DNA marker and the amount of DNA present quantified using densitometry. The amount of DNA present in the band is calculated in terms of copy numbers, as described herein.

Accordingly, in a further aspect there is provided a method for measuring mitochondrial copy number in a cell comprising the steps of: (a) amplifying a single copy nuclear gene; (b) amplifying a mitochondrial gene; (c) quantifying the amount of amplification product from step (a) and step (b); and (d) calculating the mitochondrial copy number in the cell.

In another aspect, there is provided a method for measuring mitochondrial DNA content and/or nuclear DNA content comprising the use of mass spectrophotomtery - such as MSMS.

*Expression level of one or more mitochondrial genes*

In a further embodiment, at least one indicator of altered mitochondrial activity is the expression level of one or more mitochondrial genes, wherein altered (e.g. increased) expression levels of mitochondrial gene(s) as compared to a control may be indicative of disease. One method for determining the expression level of one or more mitochondrial genes is the Nuclease Protection Assay, which may be used to detect, and quantify specific mitochondrial mRNA species. A radioactive or non-radioactively labelled single-stranded DNA (or RNA) probe is hybridised to the mitochondrial target mRNA. S1 nuclease is then used to digest all single-stranded RNA and non-duplexed probe. Under optimised conditions, S1 nuclease is highly specific for degrading single-stranded DNA and RNA while exhibiting low activity directed toward DNA or RNA in a DNA:RNA (or RNA:RNA) duplex. The remaining double-stranded hybrid is recovered by ethanol precipitation, separated by gel electrophoresis and visualised by autoradiography. Kits for performing the Nuclease Protection Assay are commercially available - such as the S1-Assay (Ambion).

Another method is RT-PCR which is both a sensitive and versatile method that can be used to determine the expression level of one or more mitochondrial genes, as described herein below. Adaptations of RT-PCR - such as real time qPCR - also allow accurate and highly sensitive quantification.

Detection of a product of enzyme catalytic activity may be accomplished directly, and in certain other embodiments detection of a product may be accomplished by introduction of a detectable reporter moiety or label into a substrate or reactant such as a marker enzyme, dye, radionuclide, luminescent group, fluorescent group or biotin, or the like. The amount of such a label that is present as unreacted substrate and/or as reaction product, following a reaction to assay enzyme catalytic activity, is then determined using a method appropriate for the specific detectable reporter moiety or label. For radioactive groups, radionuclide decay monitoring, scintillation counting, scintillation proximity assays (SPA) or autoradiographic methods are generally appropriate. For immunometric measurements, suitably labeled antibodies may be prepared including, for example, those labeled with radionuclides, with fluorophores, with affinity tags, with biotin or biotin mimetic sequences or those prepared as antibody enzyme conjugates, luminescent groups and fluorescent groups. Biotin may be detected using avidin or streptavidin, coupled to a different reporter group (commonly a radioactive or fluorescent group or an enzyme). Enzyme reporter groups may generally be detected by the addition of substrate (generally for a specific period of time), followed by spectroscopic, spectrophotometric or other analysis of the reaction products. Standards and standard additions may be used to determine the level of enzyme catalytic activity in a sample, using well known techniques.

*Mass of mitochondria*

In another embodiment, the at least one indicator of altered mitochondrial activity is the mass of the mitochondria, wherein altered (*e.g.* increased) mass of the mitochondria as compared to a control may be indicative of disease. Methods for determining the mass of mitochondria may include, for example, quantitative staining. Typically, quantitative staining of mitochondria may be performed using organelle-selective probes or dyes, including but not limited to mitochondria selective reagents - such as fluorescent dyes that bind to mitochondrial molecular components or potentiometric dyes that accumulate in mitochondria as a function of mitochondrial inner membrane electrochemical potential. Thus, by way of example, mitochondrial mass may be quantified by morphometric analysis (e.g., Cruz-Orive et al., 1990 Am. J. Physiol. 258:L148; Schwerzmann et al., 1986 J. Cell Biol. 102:97).

*Volume of mitochondria*

In another embodiment, the at least one indicator of altered mitochondrial activity is the mitochondrial volume of the mitochondria, wherein altered (*eg.* increased) mitochondrial volume as compared to a control may be indicative of disease.

Methods for determining the volume of mitochondria may include, for example, quantitative staining. Typically, quantitative staining of mitochondria may be performed using organelle-selective probes or dyes, including but not limited to mitochondria selective reagents - such as fluorescent dyes that bind to mitochondrial molecular components or potentiometric dyes that accumulate in mitochondria as a function of mitochondrial inner membrane electrochemical potential. Thus, by way of example, mitochondrial number may be quantified by morphometric analysis (e.g., Cruz-Orive et al., 1990 Am. J. Physiol. 258:L148; Schwerzmann et al., 1986 J. Cell Biol. 102:97).

*Intramitochondrial membrane potential*

In another embodiment, the at least one indicator of altered mitochondrial activity is the intramitochondrial membrane potential due to ATP synthase activity wherein altered (*eg.* increased) intramitochondrial membrane potential as compared to a control may be indicative of disease.

Delocalized lipophilic cations may be used to monitor mitochondrial membrane potential. The uptake of these cations is related to the presence of the negative sink inside the mitochondria created by the proton pump. As mitochondria increase in size due to ATP synthase or tRNA lysine defects, the transmembrane potential will increase and these defective mitochondria will accumulate lipophilic cations.

*Enzymes*

In another embodiment, the at least one indicator of altered mitochondrial activity is the activity and/or the amount of one or more enzymes involved in the electron transport chain located in the mitochondria of eukaryotic cells. Altered (eg. increased) activity and/or amount of one or more enzymes involved in the electron transport chain located in the mitochondria of eukaryotic cells as compared to a control may be indicative of disease.

The enzyme may be a terminal component of the electron transport chain located in the mitochondria of eukaryotic cells - such as cytochrome oxidase. Cytochrome oxidase (COX) is an important terminal component of the electron transport chain located in the mitochondria of eukaryotic cells. Cytochrome oxidase, also known as complex IV of the electron transport chain, is composed of at least thirteen subunits. At least ten of these subunits are encoded in nuclear genes; the remaining three subunits (I, II, and III) are encoded by mitochondrial genes.

Other methods for measuring mitochondrial activity, include, but are not limited to: (1) the oxidation-reduction sensitive dye Alamar Blue, which is a fluorometric indicator of metabolic activity (Springer et al., (1998) Brain Research Protocols Vol 2p259-263); (2) Using the dye Resazurin to detect oxidative phosphorylation without having to purify the mitochondria. This method can be used on lymphocytes using spectorphotometry (Abu-Amero and Bosley, 2005, Arch Pathol.Lab Methods Vol 129: p1295-1298); (3) Using oxygen consumption measurements with a Clarke type oxygen electrode (Hansatech LTD. Inst. London UK). This may be conducted on lymphocytes from patients. Briefly, lymphocytes are separated on a density gradient and then incubated in the presence of specific substrates and inhibitors using the electrode to measure oxygen levels (Artuch et al., 2000 Clinical Biochemistry Vol 33, no.6, p 481-485). Mitochondrial activity may also be measured using oxygen uptake measurements.

DIAGNOSIS

In one aspect, there is provided a method for identifying a subject (*eg.* a diabetic subject) having or at risk of developing disease comprising the steps of: (a) providing a test sample from said diabetic subject; and (b) measuring at least one indicator of mitochondrial activity in the test sample, wherein a difference in mitochondrial activity in the test sample as compared to a control is indicative that said subject has or is at risk of developing disease.

In a further aspect, there is provided a method for identifying a subject (*eg.* a diabetic subject) having or at risk of developing disease comprising the steps of: (a) providing a biological sample; and (b) measuring at least one indicator of mitochondrial activity in the biological sample, wherein a difference in mitochondrial activity in the biological sample as compared to a control is indicative that said subject has or is at risk of developing disease.

In a further aspect, there is provided a method for identifying a subject (eg. a diabetic subject) having or at risk of developing disease comprising the steps of: (a) providing an *in vitro* biological sample; and (b) measuring at least one indicator of mitochondrial activity in the *in vitro* biological sample, wherein a difference in mitochondrial activity in the *in vitro* biological sample as compared to a control is indicative that said subject has or is at risk of developing disease.

In one embodiment, the diagnostic method is non-invasive.

In one embodiment, the diagnostic method excludes the step of collecting the sample from the subject (*eg.* a diabetic subject).

In order to provide a basis for the diagnosis of disease, normal or standard values for mitochondrial activity should be established. This may be accomplished by measuring at least one indicator of mitochondrial activity in one or more control samples.

Accordingly, in one embodiment, a normal range of at least one indicator of mitochondrial activity is established for one or more control samples. In another embodiment, the control or standard may be a sample taken from the same patient/subject at an earlier point in time - such as before and after a patient/subject has been subjected to a particular therapeutic treatment regime.

In one embodiment, the control sample is from a subject - such as animal or human subject - that is not suffering from one or more of the diseases being tested for (*eg.* diabetes). In another embodiment, the control sample is from a subject - such as an animal or a human subject - that is suffering from one or more of the diseases being tested for (eg. diabetes) but is otherwise considered to be free from other diseases. In another embodiment, the control sample is from a subject - such as animal or human subject - that is suffering from one or more of the diseases being tested for, but wherein the sample was taken at a different (*eg.* earlier) time point (*eg.* before treatment).

The level of mitochondrial activity in a test sample may be determined (*eg*. quantified) by comparing it to one or more controls (eg. a dilution series of controls). Then, standard values obtained from one or more controls may be compared with the values obtained from one or more samples from one or more subjects affected or potentially affected by disease. Deviation between the mitochondrial activity in the one or more controls and the subject values establishes an altered mitochondrial activity and, for example, the presence or the likelihood of the subject developing one or more of the diseases described herein.

Such methods may also be tailored to evaluate the efficacy of a particular therapeutic treatment regime and may be used in animal studies, in clinical trials, or in monitoring the treatment of an individual patient. In order to provide a basis for the diagnosis of disease a normal or standard profile for mitochondrial activity should be established, as mentioned above. Deviation between the standard/control and subject values establishes the presence of disease. If disease is established, an existing therapeutic agent may be administered, and treatment profile or values may be generated.

The methods may be repeated on a regular basis to evaluate whether the values progress toward or return to the normal or standard pattern. Successive treatment profiles may be used to show the efficacy of treatment over a period of several days, several months or several years.

Those of skill in the art will appreciate that there may be any number of variations on the particular subjects, biological sources and bases for comparing levels of mitochondrial activity that are useful beyond those that are expressly presented herein.

ALTERED MITOCHONDRIAL ACTIVITY

As described herein, altered mitochondrial activity in a test sample as compared to a control is indicative that a subject has or is at risk of developing one or more of the diseases described herein.

In one embodiment, the altered mitochondrial activity is an increase in mitochondrial activity - such as at least a 2-fold increase in mitochondrial activity, at least a 3-fold increase in mitochondrial activity, at least a 4-fold increase in mitochondrial activity, at least a 5-fold increase in mitochondrial activity, at least a 6-fold increase in mitochondrial activity, at least a 7-fold increase in mitochondrial activity, at least a 8-fold increase in mitochondrial activity, at least a 9-fold increase in mitochondrial activity, at least a 10-fold increase in mitochondrial activity, at least a 20-fold increase in mitochondrial activity, at least a 25-fold increase in mitochondrial activity, at least a 50-fold increase in mitochondrial activity, at least a 100-fold increase in mitochondrial activity, or at least a 1000-fold or more increase in mitochondrial activity.

Suitably, the altered mitochondrial activity is an increase in mitochondrial activity in the test sample as compared to a control (*eg*. a healthy or a non-diseased control) which establishes the presence of, the likelihood of developing, or the susceptibility of a diabetic subject to disease.

TEST SAMPLE

The test sample may be or may be derived from a biological sample. For example, the biological sample may comprise cells or cell preparations containing mitochondria, and in certain other embodiments biological samples may comprise submitochondrial particles.

The test sample may be or may be derived from an *in vitro* sample.

Biological samples may comprise any tissue or cell preparation in which at least one indicator of mitochondrial activity can be detected, and may vary in nature depending on the particular indicator(s) to be tested.

Biological samples may be provided by obtaining a blood sample, biopsy specimen, tissue explant, organ culture (eg. kidney organ culture) or any other tissue or cell preparation (eg. a renal cell preparation and/or a mesangial cell preparation and/or lymphocytes) from a subject or a biological source.

The biological sample may be or may be derived from whole blood or a crude buffy coat fraction of whole blood, which is known in the art to comprise further a particulate fraction of whole blood enriched in white blood cells and platelets and substantially depleted of erythrocytes. The biological sample may be or may be derived from other biological fluids - such as urine.

Suitably, the test sample is nucleic acid - such as DNA and/or RNA and/or genomic DNA and/or total RNA.

Buffy coat fractions may be prepared using various methods that are known in the art - such as differential density sedimentation of blood components under defined conditions, including the use of density dependent separation media, or by other methods.

The biological sample may be or may be derived from an enriched, isolated or purified blood cell subpopulation fraction such as, for example, lymphocytes, polymorphonuclear leukocytes, granulocytes and the like. Methods for the selective preparation of particular hematopoietic cell subpopulations are well known in the art (see, e.g., Current Protocols in Immunology, J. E. Coligan et al., (Eds.) 1998 John Wiley & Sons, NY).

The subject or the biological source of the test sample may be a human or non-human animal, a primary cell culture or culture adapted cell line including but not limited to genetically engineered cell lines that may contain chromosomally integrated or episomal recombinant nucleic acid sequences, immortalized or immortalizable cell lines, somatic cell hybrid, differentiated or differentiable cell lines, transformed cell lines and the like.

Cytoplasmic hybrid "cybrid" cell lines using mitochondria from either control subjects or subjects having or suspected of being at risk of disease may be prepared. Such cybrids may be used to determine levels of mitochondrial activity for diagnostic or predictive purposes, or as biological sources for screening assays to identify agents that may be suitable for treating disease based on their ability to alter the levels of mitochondrial activity in treated cells.

In one specific embodiment, the test sample is a cultured cell - such as a cultured renal cell. Suitably, the cultured renal cell is a cultured mesangial cell.

In one embodiment, the subject may be suspected of having or be at risk of having, or have a disease resulting from oxidative stress in the body.

In one embodiment, the subject may be suspected of having or be at risk of having, or have diabetes mellitus.

In one embodiment, the subject may be suspected of having or be at risk of having, or have type 1 diabetes mellitus.

In one embodiment, the subject may be suspected of having or be at risk of having, or have type 2 diabetes mellitus.

The requirements for meeting the clinical criteria for having or being at risk of having type 2 diabetes mellitus is described in Gavin et al. Diabetes Care 22 (suppl.1):S5-S19, 1999, American Diabetes Association Expert Committee on the Diagnosis and Classification of Diabetes Mellitus.

Such determinations may have prognostic and/or diagnostic usefulness.

Where it is desirable to determine whether or not a subject or biological source falls within clinical parameters that are indicative of disease, signs and symptoms of disease that are accepted by those skilled in the art may be used to so designate a subject or biological source as suffering from disease.

The subject or biological source may be suspected of having or being at risk for having disease, and in certain embodiments the subject or biological source may be known to be free of a risk or presence of such a disease.

MODULATING

As used herein, the term "modulates" refers to preventing, suppressing, alleviating, restorating, elevating or otherwise affecting at least one indicator of altered mitochondrial activity.

In the context of agents that modulate disease, it is preferred that "modulate" refers to preventing, suppressing or alleviating disease.

AMPLIFICATION

As used herein, "amplification" refers to any process for multiplying strands of nucleic acid - such as genomic DNA - *in vitro.* Suitably, the process is enzymatic and is linear or exponential in character.

Amplification techniques include, but are not limited to, methods broadly classified as thermal cycling amplification methods and isothermal amplification methods. Suitable thermal cycling methods include, for example, ligase chain reaction (Genomics 4:560, (1989); and Science 241: 1077 (1988)). The ligase chain reaction uses DNA ligase and four oligonucleotides, two per target strand. The oligonucleotides hybridise to adjacent sequences on the nucleic acid to be amplified and are joined by the ligase. The reaction is heat denatured and the cycle repeated. Isothermal amplification methods useful in the present invention include, for example, Strand Displacement Amplification (SDA) (Proc. Nat. Acad. Sci. USA 89:392-396 (1992)), Q-beta-replicase (Bio/Technology 6:1197-1202 (1988)); nucleic acid-based Sequence Amplification (NASBA) (Bio/Technology 13:563-565 (1995)); and Self-Sustained Sequence Replication (Proc. Nat. Acad. Sci. USA 87:1874-1878 (1990)).

A particularly suitable amplification method is PCR. As is well known to a person skilled in the art, this is a method in which virtually any nucleic acid sequence can be selectively amplified. The method involves using paired sets of oligonucleotides of predetermined sequence that hybridise to opposite strands of DNA and define the limits of the sequence to be amplified. The oligonucleotides prime multiple sequential rounds of DNA synthesis catalysed by a thermostable DNA polymerase. Each round of synthesis is typically separated by a melting and re-annealing step, allowing a given DNA sequence to be amplified several hundred-fold in less than an hour (Science 239:487, 1988). As described herein, more than one nucleic acid can be simultaneously amplified by multiplex PCR in which multiple paired primers are employed.

One suitable PCR based method is quantitative PCR that may be preformed using, for example, the Roche Light Cycler.

In one embodiment, high throughput PCR is used such that at least 96 samples or at least 384 samples can be processed at the same time - using for example, the ABI Prism 7000 or the ABI 9700 analysis system, respectively.

In particular, one method is the primer extension assay disclosed by Fahy et al. (Nucl. Acids Res. 25:3102, 1997) and by Ghosh et al. (Am. J. Hum. Genet. 58:325, 1996). Hybridization conditions may be varied according to the particular nucleic acid target and oligonucleotide probe selected, using methodologies well known to those having ordinary skill in the art.

Other useful amplification techniques include, for example, ligase chain reaction (e.g., Barany, Proc. Nat. Acad. Sci. 88:189, 1991), Q-beta replicase assay (Cahill et al., Clin. Chem. 37:1482, 1991; Lizardi et al., Biotechnol. 6:1197, 1988; Fox et al., J. Clin. Lab. Analysis 3:378, 1989) and cycled probe technology (e.g., Cloney et al., Clin. Chem. 40: 656, 1994), as well as other suitable methods that will be known to those familiar with the art.

Oligonucleotides for use in the present invention may be produced recombinantly, synthetically, or by any means available to those of skill in the art. They may also be cloned by standard techniques. In general, primers will be produced by synthetic means, involving a stepwise manufacture of the desired nucleic acid sequence one nucleotide at a time. Techniques for accomplishing this using automated techniques are readily available in the art. Longer nucleotide sequences will generally be produced using recombinant means, for example using PCR (polymerase chain reaction) cloning techniques. This will involve making a pair of primers (e.g. of about 15 to 30 nucleotides) flanking a region of the targeting sequence which it is desired to clone, bringing the primers into contact with mRNA or cDNA obtained from an animal or human cell, performing a polymerase chain reaction (PCR) under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector.

Suitably, at least one set of primers that are used in the amplification reaction will specifically hybridise to one or more mitochondrial genes situated in mitochondrial DNA (mtDNA).

Suitably, at least one set of primers that are used in the amplification reaction will specifically hybridise to one or more genes of known copy number - such as one or more nuclear genes - and so the copy number of the one or more mitochondrial genes can be determined.

RT-PCR

As described herein, RT-PCR may be used to determine the expression level of one or more mitochondrial genes. Since RNA cannot serve as a template for PCR, reverse transcription is combined with PCR to make complementary DNA (cDNA) suitable for PCR. This combination of both technologies is referred to as RT-PCR. Various types of primers may be used for reverse transcription - such as oligo (dT)12-18 which binds to the poly (A) tail at the 3' end of mammalian RNA; random hexanucleotides which bind to mRNA at any complementary site and may be better for overcoming difficulties caused by template secondary structure; and specific oligonucleotide primers can be used to selectively prime the mitochondrial RNA sequence of interest. Selection of an appropriate primer for reverse transcription is dependent upon the size and secondary structure of the mitochondrial mRNA.

RT-PCR may be one-tube or two-tube. In one-tube RT-PCR, both reverse transcription and PCR are performed in the same tube. A thermostable DNA polymerase - such as Taq DNA polymerase - and all necessary primers and reagents are added during the reaction set-up. cDNA is synthesised by reverse transcription at 37°C while the DNA polymerase in the reaction mix has little activity. After allowing sufficient time for reverse transcription, the temperature is raised, inactivating the reverse transcriptase and allowing the DNA polymerase to amplify the cDNA. In two-tube RT-PCR, cDNA is first synthesised by reverse transcription. An aliquot of the finished reverse-transcription reaction is then used for PCR. Kits for performing RT-PCR are commercially available - such as the Polymerase One-Step RT-PCR System (Roche Molecular Biochemicals), Titan One-Tube RT-PCR Kit (Roche Molecular Biochemicals) and OneStep RT-PCR Kit (Qiagen).

HYBRIDISATION

The term "hybridisation" as used herein shall include "the process by which a strand of nucleic acid joins with a complementary strand through base pairing" as well as the process of amplification as carried out in, for example, PCR or RT-PCR.

ASSAY

Advantageously, there are also provided methods for screening compounds to identify agents that modulate disease.

For example, one or more mitochondria may be contacted with an agent. The ability of the agent to modulate the activity of the mitochondria following interaction with the agent is then measured.

The screening methods may comprise the steps of mixing an agent with a solution containing one or more mitochondria, to form a mixture, and determining whether its ability to modulate the activity of the mitochondria is modulated.

Thus, in certain embodiments, the one or more mitochondria may be used to screen for agents - such as antibodies or peptides - that modulate mitochondrial activity, thereby identifying a therapeutic agent capable of modulating mitochondrial activity. For example, anti- mitochondrial antibodies capable of modulating the activity of the mitochondria may be used to modulate mitochondrial activity. Alternatively, screening of peptide libraries or organic libraries made by combinatorial chemistry may be useful for identification of therapeutic agents that function by modulating mitochondrial activity. Synthetic compounds, natural products, and other sources of potentially biologically active materials can be screened in a number of ways deemed to be routine to those of skill in the art.

The assay method of the present invention may be a high throughput screen (HTS).

In certain other embodiments, biological samples containing at least one indicator of altered mitochondrial activity may be obtained from a subject or biological source before and after contacting the subject or biological source with an agent, for example to identify an agent capable of effecting a change in the level of the indicator of altered mitochondrial activity, relative to the level before exposure of the subject or biological source to the agent. Thus, by way of example, if the indicator is an enzyme, the mitochondrial activity may be determined as a measure of enzyme activity in the sample, as a measure of enzyme quantity in the sample or as a measure of enzyme expression level in the sample.

Candidate agents further may be provided as members of a combinatorial library, which preferably includes synthetic agents prepared according to a plurality of predetermined chemical reactions performed in a plurality of reaction vessels. For example, various starting compounds may be prepared employing one or more of solid-phase synthesis, recorded random mix methodologies and recorded reaction split techniques that permit a given constituent to traceably undergo a plurality of permutations and/or combinations of reaction conditions. The resulting products comprise a library that can be screened followed by iterative selection and synthesis procedures, such as a synthetic combinatorial library of peptides or other compositions that may include small molecules as provided herein (see e.g., EP 0774464, U.S. Pat. Nos. 5,798,035, 5,789,172, 5,751,629). Those having ordinary skill in the art will appreciate that a diverse assortment of such libraries may be prepared according to established procedures, and tested for their influence on an indicator of altered mitochondrial activity.

The candidate agent may be or may be derived from an agent that is known to modulate mitochondrial activity - such as anti-retroviral drugs (e.g. DDC) - or thiazolidenedione based agents which affect mitochondrial biogenesis (Bagocka et al., 2005 Diabetes 54:1392-1399,).

There are also provided compositions and methods that are useful in pharmacogenomics, for the classification and/or stratification of a subject or patient population. In one embodiment, for example, such stratification may be achieved by identification in a subject or patient population of one or more distinct profiles of at least one indicator of mitochondrial activity that correlates with disease. Such profiles may define parameters indicative of a subject's predisposition to develop disease, and may further be useful in the identification of novel subtypes of disease.

As described herein, determination of levels of at least one indicator of mitochondrial activity may also be used to stratify a disease patient population (*i.e.*, a population classified as having disease by independent criteria). Accordingly, in another embodiment, determination of levels of at least one indicator of mitochondrial activity in a biological sample from a subject may provide a useful correlative indicator for that subject. A subject so classified on the basis of levels of at least one indicator of mitochondrial activity may be monitored using disease clinical parameters referred to above, such that correlation between levels of at least one indicator of mitochondrial activity and any particular clinical score used to evaluate disease may be monitored.

For example, stratification of a disease patient population according to levels of at least one indicator of mitochondrial activity may provide a useful marker with which to correlate the efficacy of any candidate therapeutic agent being used in diabetic subjects suffering from disease.

In another embodiment, correlation of one or more traits in a subject with at least one indicator of altered mitochondrial activity may be used to gauge the subject's responsiveness to, or the efficacy of, a particular therapeutic treatment.

In certain embodiments, the invention provides a method of treating a patient having disease by administering to the patient an agent that substantially restores at least one indicator of altered mitochondrial activity to a level found in control or normal subjects.

In one embodiment, an agent that substantially restores (*e.g.*, increases or decreases) at least one indicator of mitochondrial activity to a normal level effects the return of the level of that indicator to a level found in control subjects.

In another embodiment, the agent that substantially restores such an indicator confers a clinically beneficial effect on the subject.

In another embodiment, the agent that substantially restores the indicator promotes a statistically significant change in the level of at least one indicator of mitochondrial activity.

A person skilled in the art can readily determine whether a change in the level of a particular indicator brings that level closer to a normal value and/or clinically benefits the subject. Thus, an agent that substantially restores at least one indicator of mitochondrial activity to a normal level may include an agent capable of fully or partially restoring such level.

GENES

In a further aspect, the expression profile of one or more mitochondrial genes in a test sample is determined in order to identify one or more gene(s) that are involved in the presentation of disease.

The expression profile of one or mitochondrial genes may be determined using various methods known in the art, for example, Northern blotting, *in situ* hybridisation, nuclease protection assay and RT-PCR.

Northern blotting is a technique for size fractionating RNA in a gel, followed by transfer and immobilisation on a solid support, for example, a membrane in such a manner that the relative positions of the RNA molecules are maintained. The resulting Northern blot is then hybridised with a labelled probe complementary to the mRNA of interest. Signal generated from detection of the probe can be used to determine the size and abundance of the target RNA. Northern blotting analysis may be performed using total RNA and a radioactively labelled mitochondrial gene fragment. The steps involved in Northern analysis include: RNA isolation (total or poly(A) RNA), probe generation, denaturing agarose gel electrophoresis, transfer to solid support and immobilization, prehybridisation and hybridization with probe, washing, detection and finally stripping and reprobing (optional). Kits for practising Northern blotting are commercially available such as the NorthernMax Kit (Ambion).

*In situ* hybridisation (ISH) may be used. Various probes may be used in this method - such as RNA probes, which may be generated and labelled by *in vitro* transcription procedures or synthetic oligonucleotide probes which may be prepared using various methods known in the art e.g. by automated chemical synthesis. Oligonucleotide probes are labelled during synthesis or by addition of reporter molecules at the 5' or 3' end after synthesis. Probes may be labelled in various ways, using radioactive labels e.g. $^{32}$P, or non-radicoactibe labels e.g. DIG. The ISH method comprises a number of steps as briefly outlined below:

i. Prehybridisation - Samples e.g. sections of tissues, to be hybridised are incubated with phosphate buffered saline (PBS), washed and then permeabilised with buffer containing either RNase-free Proteinase K or RNase-free Proteinase K. Samples are post-fixed with PBS containing para-formaldehyde and washed with PBS. Samples are acetylated with TEA buffer containing acetic anhydride and incubated with prehybridisation buffer.

ii. *In situ* hybridisation - A hybridisation buffer is prepared and slides are dipped briefly in distilled water. Prehybridisation buffer is drained from the slides and overlayed with the hybridisation buffer containing labelled probe. Samples are covered and incubated overnight in a humid chamber.

iii Posthybridisation - Coverslips are removed and samples washed. Unbound RNA probe is removed and the samples again washed.

iv Immunological detection - Samples are washed with buffer and covered with blocking solution, followed by incubation in a humid chamber with a suitable dilution of labelled antibody - such as sheep anti-DIG-alkaline phosphatase. Samples are again washed and a colour solution prepared containing, for example, nitroblue tetrazolium solution, 5-bromo-4-chloro-3-indolyl-phosphate and levamisole. Each sample is covered with the colour solution and slides are incubated in a humid chamber in the dark. When colour development is optimal, the color reaction is stopped and slides are dipped briefly in distilled water. Samples are counterstained and washed.

Additional information concerning *in situ* hybridisation is available in Komminoth (1992) Diagn. Mol. Pathol. 1, 142-150, Komminoth et al. (1992) Histochemistry 98, 217-228 and Sambrook et al. Molecular Cloning, A Laboratory Manual (1989).

Still other methods that can be used are the Nuclease Protection Assay and RT-PCR as already described herein.

It will be understood by a skilled person that other methods can be used to determine the expression of one or more mitochondrial genes.

TREATMENT OF DISEASE

Using the methods described herein, a subject may be diagnosed with or be at risk of developing one or more of the diseases described herein.

Accordingly, the subject may be treated to slow or halt the progression or development of disease(s).

For example, current treatment strategies directed at slowing the progression of diabetic nephropathy include optimized glycemic control (through modification of diet and/or insulin therapy) and hypertension control. For example, both angiotensin-converting enzyme (ACE) inhibitors and angiotensin receptor blockers (ARBs), administered to reduce hypertension, have been shown to delay progression or development of nephropathy and macroalbuminuria. Several clinical trials have established the benefits of ACE inhibitors and ARBs in patients with diabetes.

Other treatment strategies have focused on one or more growth factors as therapeutic targets. Therapies directed at inhibiting VEGF or TGF.beta., either alone or in combination with ACE inhibitors or ARBs, have been examined. (See, e.g., De Vriese et al. (2001) J. Am Soc Nephrol 12:993-1000; Flyvbjerg et al. (2002) Diabetes 51:3090-3094; Ziyadeh et al, (2000) Proc Natl Acad Sci 97:8015-8020; Chen et al. (2003) Biochem Biophys Res Commun 300:16-22; and Benigni et al. (2003) J. Am Soc Nephrol 14:1816-1824).

A review of the treatment and prevention of diabetic nephropathy can be found in Int. J. Urol & Nephrol. (2005) 37, 655-663.

GENERAL RECOMBINANT DNA METHODOLOGY TECHNIQUES

The present invention employs, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; and, D. M. J. Lilley and J. E. Dahlberg, 1992, Methods of Enzymology: DNA Structure Part A: Synthesis and Physical Analysis of DNA Methods in Enzymology, Academic Press. Each of these general texts is herein incorporated by reference.

The invention will now be further described by way of Examples, which are meant to serve to assist one of ordinary skill in the art in carrying out the invention and are not intended in any way to limit the scope of the invention.

EXAMPLES

INTRODUCTION

As described herein, the inventor has surprisingly discovered that mitochondrial numbers may be used as a diagnostic tool to predict the risk of developing disease - such as a disease resulting from oxidative stress in the body - such as nephropathy (eg. diabetic nephropathy).

Diabetic nephropathy (DN) is progressive disease which affects 30 to 50% of patients with diabetes mellitus and is a major cause of end stage renal disease in the western world [1-3]. DN takes several years to develop and currently the first clinical indicator is microalbuminuria, the appearance of albumin, a common blood protein, in the urine. Microalbuminuria has also become accepted as a predictor of cardiovascular dysfunction in recent years [4].

The mechanisms involved in the development and progression of DN are not fully understood. High plasma glucose levels lead to increased cytosolic glucose concentrations in renal cells with consequent biochemical dysfunction such as the abnormal activation of various signalling pathways and oxidative stress [6,7,8].

The production of reactive oxygen species has been recently postulated to be a central mechanism to activate the pathways that lead to the damage found in the complications of diabetes [5]. Much of this damage is thought to be a consequence of increased production of ROS by the mitochondrial respiratory chain during hyperglycemia. Electron leakage during the conversion of ADP to ATP via the electron transport chains located in the mitochondria results in increased ROS production and can lead to direct damage to DNA, lipids and proteins and also indirectly via alterations in cellular redox which can consequently affect signalling in the cell via thiol dependent proteins [8,9]. The pathways triggered can then lead to accumulation of extra cellular matrix, leading to glomerulosclerosis, the pathological hall mark of DN [6].

It is well accepted that glucose plays a significant role in the development of DN [11,12], and may cause some long term changes in those patients who go on to develop complications. One puzzling aspect of DN and indeed several other types of nephropathy (for example nephropathy associated with hypertension), is that only a subset, say 30-50% of patients develop it, whereas the rest do not. The question arises as to what are the differences between those that develop nephropathy and those that do not. There is some evidence to show that some of the predisposition may be due to genetic factors [10], however this is likely to be only a small percentage of those affected.

Elevated mitochondrial numbers may provide an explanation for this phenomenon as it predicts that patients predisposed to nephropathy develop a "permanent" change - such as enhanced mitochondrial numbers leading to enhanced mitochondrial activity which in turn would lead to increased leakage of reactive electrons from the miotchondrial electron transport chains. This may increase the intracellular reactive oxygen species levels resulting in abnormal activation of pathways which trigger the pathological changes associated with nephropathy. It is possible that most body cells show increased mitochondrial activity in response to high glucose or certain other triggers (e.g oxidised LDL) and that the impact is only seen in the organs and tissues of the body which are more predisposed to oxidative stress damage, *e.g.* the kidney, the eye, the cardiovascular system and/or the neural system.

EXAMPLE 1

*Materials and methods*

Human mesangial cells: The human mesangial cell line used in this study was obtained from Dr Nadia Wahab (School of Medicine, Imperial College London). HMCL cells were grown to confluence, growth arrested, and seeded in triplicate at a density of $2 \times 10^5$ cells, using 2 different experimental conditions: normal glucose (5mM), and high glucose (25mM) for 4 days. Cells were detached using trypsin and then either used directly for genomic DNA or RNA preparation or frozen as a pellet at -80'C until required.

Patients' blood genomic DNAs: Genomic DNA extracted from blood samples of patients with diabetic nephropathy, patients with diabetes and no nephropathy, and healthy controls, were obtained from Dr Iqbal Masud (National Institute of Kidney Diseases & Urology, Birdem Hospital, Sher-e -Bagla Nagar 120, Dhaka, BANGLADESH) and were stored either as working solutions at 4'C or at -20'C as stocks.

Rat kidney samples: GK rat and Wistar rat kidneys obtained through a collaboration with Dr John Williams, (Institute of Nephrology, University of Wales College of Medicine. All rat kidney tissues were stored at -80'C.

RNA/DNA Extraction. Total cellular RNA was extracted from tissues using the Totally RNA kit (Ambion Inc.) or from cells using RNAqueous-4PCR kit (Ambion Inc.) as previously described (18). Genomic DNA extraction from the tissues were performed using GenomicPrep Cells and Tissue DNA Isolation kit (Amersham Biosciences, UK) as described by the manufacturer.

cDNA preparation

10 μg of RNA was mixed with 0.5 μl oligodT primers (Ambion), heated (70 °C, 5 min), rapidly cooled on ice, and a reaction mixture consisting of 5 μl of 5X RT Buffer, 2.5 μl of 20 mM dNTP mix, 1 μl AMV reverse transcriptase (30u/μl) and DEPC-treated water to make up a total volume of 25 μl was added. After incubation (42 °C, 1 hr, 95 °C, 5 min), the cDNAs were stored at -20°C until usage. For RT-PCR 0.1 to 0.25μl of cDNA was used as a template. For real time PCR 2μl of a 1 in 10 dilution of cDNA was used as a template.

Oligonucleotide primers

In order to amplify the coding regions of the genes used in this study, oligonucleotide primers of about 100-400 bp long were designed using Primer3 software at http://frodo.wi.mit.edu/cgi-bin/primer3/primer3_www.cgi. Primers were tested using normal or gradient Polymerase Chain Reaction (PCR) to find the optimal conditions for each set of primers. A full list of primers used in this study is described herein.

Polymerase Chain Reaction (PCR)

PCR was carried out in PCR tubes (200μl) by adding and mixing 5μl of Mg free buffer, 1μl of 2.5mM dNTPs, 1μl of forward primer, 1μl of reverse primer, optimal concentration of $MgCl_2$ (usually 3mM), 1μl of the DNA template, 0.6μl of Taq polymerase and DEPC treated water was added to reach the final volume of 50μl The reaction was carried out in the PCR thermal cycler (GeneAmp PCR System 2400, Applied Biosystem, UK) or in the gradient PCR machine (DNA Engine, DYAD™, UK). The usual conditions for PCR were initial denaturation (94°C, 15min), and then 30 cycles of denaturation (94°C, 30sec), annealing (primer annealing temp, 30sec), (elongation 72°C, 90sec), followed by final elongation (72°C, 7min) and then storage at 4°C until usage. The PCR products were run on an agarose gel (1% in TBE) with a size marker (100bp or 1Kb) to identify the size of the amplified PCR product.

PCR products were purified using GFX™ PCR DNA and Gel Band Purification Kit (Amersham Biosciences, UK).

Densitometry of purified PCR products

To calculate the amount of DNA in the sample of purified PCR products, 5μl of each sample and 100 bp DNA ladder mixed with 3 μl of the loading buffer were electrophoresed in a 1% Agarose gel in 1x TBE buffer containing ethidium bromide. The gels were visualized under UV light and the image was digitized and printed using a digital imaging system called Alpalmager 2000 (Alpha Innotech Corp., San Leandro, CA). 5 $\mu$l of the 100bp or 1 Kb DNA ladder mixed with 2 $\mu$l of the loading buffer was also loaded on the gel in order to determine the size of each band. The 500bp band from the 100 bp ladder, known to contain 150ng of DNA was used to calculate the amount of DNA in the PCR product band (copies of each gene) (the calculation is shown in the result section).

Preparation of serial dilutions (Standards)

The identity of each PCR product used to generate standards was confirmed using DNA sequencing with the appropriate primers. The amount of DNA present was determined by electrophoresis of the PCR products against the 100bp DNA ladder [Promega Corporation] using densitometry with the UVP DNA imaging system. A dilution series containing $10^7$, $10^6$, $10^5$, $10^4$, $10^3$, and $10^2$ copies per ul of each gene was prepared in the presence of carrier tRNA [Sigma] and used as a calibration curve. Serial dilution was carried out in order to provide standards of known copy number for each gene being quantitated. The copies of DNA per $\mu$l were calculated by densitometry and a calculation was performed (see results). Transfer ribonucleic acid (tRNA) was used as a carrier and nuclease free DEPC treated water was used to make the final volume of 100 $\mu$l for each standard.

Quantitative PCR

Real-time qPCR reactions were carried out in a total volume of 10 ul. The reaction mixture contained forward and reverse primers, FastStart DNA Master SYBR Green I [Roche Molecular Biochemicals], DNA template and nuclease free water [Ambion, UK]. Quantification of gene copy numbers in different samples was carried out in the presence of appropriate dilution series. Each biological sample was obtained at least in duplicate and usually in triplicate and qPCR was carried out in duplicate on each of these, with resulting values being averaged. The reactions were performed in the Roche lightcycler using the following 4-cycle program protocol: pre-incubation at 95°C for 10 min; amplification at 95°C for 10 sec, variable annealing temperatures [Table 1] for 20 sec and 72 °C for 8-17 sec [extension time = product size [bp]/25] for 40 cycles; melting curve at 95 °C for 0 sec, 65 °C for 30 sec and 95 °C for 0sec; cooling at 40 °C for 30 sec. The resulting data was analyzed using the Roche Molecular Biochemicals lightcycler software, version 3.5.

EXAMPLE 2

*Altered mRNA expression of mitochondrial genes in the kidneys of the GK rat*

The GK rat is normo-glycemic at 6 weeks (6mM blood glucose) and by 26 weeks is hyper-glycemic [14-18], with hyperglycemia developing progressively. Differential display and screening was used to isolate a number of genes that showed changes in mRNA levels between 6 weeks and 26 weeks in the kidneys of the GK rat [14,21]. A list of some of the clones isolated is shown in Table 1. It was surprising that nearly 40% of the clones isolated on the basis of altered renal mRNA expression turned out to encode mitochondrial proteins, suggesting that hyperglcyemia in the GK rat is accompanied by altered mitochondrial activity.

The elevated expression of some of the mitochondrial clones we isolated was confirmed using qPCR. For example, we showed that the NDUFA10 gene of the NADH ubiquinone oxidoreductase complex 1, part of the mitochondrial electron transport chain located in the inner mitochondrial membrane, is up-regulated >3fold at the mRNA level in diabetic kidneys of the GK rat compared to nonnoglycemic control rats (data not shown).

The increased expression of a nuclear-encoded mitochondrial mRNA suggests increased mitochondrial biogenesis in diabetic kidneys. If this is so then the mitochondrial encoded mRNAs would also need to be up-regulated. One level of regulation of mitochondrial mRNAs is increased copy numbers of the mitochondrial genome which can lead to enhanced expression of mitochondrially encoded genes. Therefore, mitochondrial activity (*eg*. mitochondrial genome copy numbers) may be enhanced in response to hyperglycemia to allow increased transcription of the mitochondrially encoded mRNAs to match the levels of nuclear encoded mitochondrial mRNAs such as NDUFA10.

EXAMPLE 3

*An assay for measuring mitochondrial copy numbers in rat and human cells*

An assay based on real time PCR to measure exact mitochondrial genome copy numbers in rat and human was designed. In a normal non-dividing cell, there is a single nuclear genome whereas there are hundreds to thousands of copies of the mitochondrial genome. We designed primers to a single copy nuclear gene and a single mitochondrial gene. In the case of rat the nuclear gene used was either actin or B2microglobulin (rB2M), and the mitochondrial gene was cytochrome B (rCYTB,). For human cells and patients we used hCYTB and hB2M, the latter a single copy gene.

To prepare standards each gene was amplified using the specific primers, the PCR products were then electrophoresed against a known DNA marker and the amount of DNA present was quantified using densitometry. The amount of DS DNA present in the band was calculated in terms of copy numbers using the following equation

$$\text{Copies of gene} = \frac{\text{Avagadro's number x concentration of DNA (g/ul)}}{660 \text{ x length of template DNA}}$$

(Avagadro's number $= 6.02 \times 10^{23}$, m.wt. of DNA $= 660$)

A dilution series was prepared containing $0.5 \times 10^7$, $0.5 \times 10^6$, $0.5 \times 10^5$, $0.5 \times 10^4$, $0.5 \times 10^3$, $0.5 \times 10^2$, copies of plasmid DNA per ul.

EXAMPLE 4

*Increased mitochondrial copy numbers in diabetic rat kidneys*

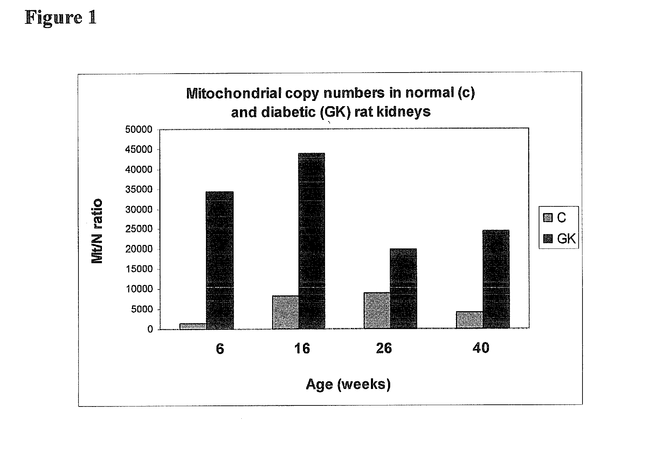

We quantified mitochondrial genome copy numbers per nuclear genome in the kidneys of control Wistar rat (C) and diabetic GK rat (GK) at different ages (6, 16, 26 and 40 weeks). Genomic DNA was extracted from control and diabetic kidneys and real time PCR was used to quantify r-Actin, and r-CYTB. The ratio of r-CYTB to actin was calculated. As actin is a single copy gene, the results can be interpreted as copy numbers of mitochondria versus nuclear genome. The results are shown in Figure 1.

As shown in Figure 1, the mitochondrial to nuclear genome ratio is considerably higher in the GK rat compared to the Wistar rats at all the ages examined. These data show that the diabetic rat kidneys contain more mitochondria per cell than the control rat kidneys.

The demonstration of increased mitochondria in the GK rat kidneys suggests that hyperglycemia may be the cause of increased mitochondrial biogenesis in the GK rat

EXAMPLE 5

*High glucose results in increased copy numbers of mitochondria in human RENAL cells*

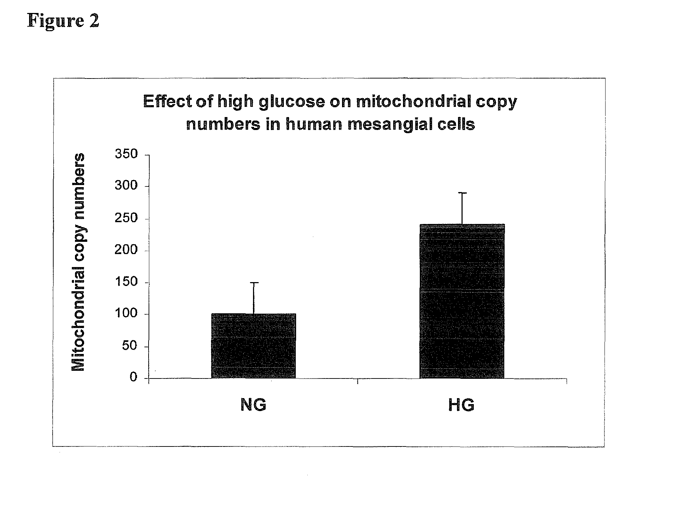

To test the hypothesis that glucose can directly increase mitochondrial numbers, we cultured human mesangial cells (HMCs) in normal and high glucose in triplicate. Genomic DNA was extracted from the HMCs and the copy numbers of hCYTB and hActin were measured using real time qPCR. The ratio of hCYTB to hActin was calculated to determine the mitochondrial copy numbers per cell. The results are shown in Figure 2.

Exposure to 25mM glucose appears to lead to a significant increase (>200%) in mitochondrial genome copy numbers, suggesting the glucose can directly result in enhanced mitochondrial biogenesis.

EXAMPLE 6

*Patients with diabetic nephropathy have elevated mitochondrial copy numbers*

Genomic DNA was extracted from blood samples of diabetics with nephropathy (DN, 19 samples shown in black), diabetics without nephropathy (DM, 6 samples, shown in red) ) and healthy controls (HC, 19 samples), and used to measure the ratio of hCYTB to hActin in order to determine the mitochondrial copy numbers in blood. The data show the same trend as that seen in diabetic kidneys and cultured mesangial cells, *i.e* that patients with DN have an enhanced mitochondrial copy number.

The results are shown in Figure 3.

As can be seen, in most cases the patients with diabetic nephropathy have an enhanced mitochondrial genome copy number when compared with the two sets of controls. Using the statistical package SPSS we determined the mean values, the confidence intervals (i.e. the range of values) and the standard error for the mitochondrial to nuclear genome ratio for the 3 groups (Table 2).

These results support the finding that in patients with diabetic nephropathy there is an enhanced mitochondrial to nuclear genome ratio.

CONCLUSIONS

Rat diabetic kidneys have a greater mitochondrial to nuclear genome ratio compared with normo-glycemic rat kidneys.

Glucose can directly enhance mitochondrial copy numbers in cultured human renal (mesangial) cells.

From the group of samples studied, most patients with diabetic nephropathy have a significantly higher mitochondrial to nuclear genome ratio when compared with healthy controls and with diabetics without nephropathy.

SUMMARY

Using differential screening, several mitochondrial genes were up-regulated at the mRNA level in the kidney during progressive hyperglycemia in the GK rat (Table 1). Using real time qPCR, progressive increase in the mRNA of a nuclear-encoded gene which encodes a mitochondrial protein was confirmed, this increase correlates with progressive hyperglycemia. Increased expression of nuclear encoded mitochondrial genes implies increased mitochondrial biogenesis. To test this, an assay was designed to measure mitochondrial genome copy numbers using a real time PCR assay in both rat and human. With this method the Mt/N ratio (mitochondrial genome relative to nuclear genome) i. e. mitochondrial genome numbers per cell was determined.

Data showing increased mitochondrial genome copy numbers:

a) Diabetic rat kidneys have a significantly higher Mt/N ratio compared to age-matched normal kidneys.

b) Cultured human glomerular mesangial cells show a 200% increase in mitochondrial copy numbers after 4 days of growth in high glucose.

c) Mitochondrial copy numbers are significantly enhanced in patients with DN when compared with diabetics with no nephropathy and healthy controls.

In summary, it is shown herein for the first time that hyperglycemia results in a significant alteration (*eg*. increase) in mitochondrial copy numbers in renal cells, both *in vitro* and *in vivo,* and patients with DN show increased mitochondrial copy numbers in blood samples. Without being bound by any particular theory, increased mitochondrial activity could result in increased production of reactive oxygen species via enhanced electron leakage from mitochondrial electron transport, thereby leading to the damage. In some patients this increase in mitochondrial copy numbers could be long term or permanent, leading to continued enhanced ROS production in cells (*eg*. renal cells) over time leading to the development of disease - such as DN. Mt/N ratio could be used to predict patients at risk of developing a disease resulting from oxidative stress in the body - such as DN and other complications of diabetes, and also to monitor their chances of recovery.

FURTHER ASPECTS

Further aspects are presented in the following numbered paragraphs.

1. A method for identifying a diabetic subject having or at risk of developing microvascular disease, kidney disease and/or cardiovascular disease comprising the steps of:

(a) providing a test sample from said diabetic subject; and

(b) measuring at least one indicator of altered mitochondrial activity in the test sample,

wherein a difference in mitochondrial activity in the test sample as compared to a control is indicative that said subject has or is at risk of developing said disease(s).

2. The method according to paragraph 1, wherein the difference in mitochondrial activity is an increase in mitochondrial activity.

3. The method according to paragraph 1 or paragraph 2, wherein the at least one indicator of altered mitochondrial activity is the number of mitochondria in the test sample as compared to the control.

4. The method according to paragraph 3, wherein the number of mitochondria is measured using oxygen uptake measurements, measuring cytochrome oxidiase activity and/or counting the number of mitochondria using a microscope.

5. The method according to paragraph 1 or paragraph 2, wherein the at least one indicator of altered mitochondrial activity is the copy number of the mitochondrial genome in the test sample as compared to the control.

6. The method according to paragraph 5, wherein the copy number of the mitochondrial genome is determined by comparing it to the copy number of the nuclear genome.

7. The method according to paragraph 5 or paragraph 6, wherein the copy number is determined using PCR amplification.

8. The method according to any of paragraphs 5 to 7, wherein the copy number is determined using real-time PCR amplification.

9. The method according to any of the preceding paragraphs, wherein the test sample is or is derived from a blood sample, a biopsy specimen, a tissue explant, an organ culture or a cultured cell.

10. The method according to any of the preceding paragraphs, wherein the test sample is nucleic acid that is or is derived from a blood sample, a biopsy specimen, a tissue explant, an organ culture or a cultured cell.

11. The method according to paragraph 10, wherein the nucleic acid is genomic DNA and/or total RNA.

12. The method according to any of paragraphs 9 to 11, wherein the cultured cell is a cultured renal cell.

13. The method according to the paragraph12, wherein the cultured renal cell is a mesangial cell or a tubular cell.

14. The method according to any of paragraphs 9 to 13, wherein said cultured cell is grown in high or low glucose prior to measuring the at least one indicator of mitochondrial activity.

15. The method according to any of the preceding paragraphs, wherein the microvascular disease is diabetic nephropathy, diabetic retinopathy and/or diabetic neuropathy.

16. The method according to any of paragraphs 1-14, wherein the kidney disease is kidney disease associated with hypertension, kidney disease associated with metabolic syndrome, kidney disease associated with gloemrulonephritis and/or cardiovascular disease.

17. A method for measuring mitochondrial copy number in a cell comprising the steps of:

   (a) amplifying a single copy region of the nuclear genome;
   (b) amplifying a region of the mitochondrial genome;
   (c) quantifying the amount of amplification product from step (a) and step (b); and
   (d) calculating the mitochondrial copy number in the cell.

18. The method according to paragraph 17, wherein the single copy region of the nuclear genome is a single copy nuclear gene.

19. The method according to paragraph 17 or paragraph 18, wherein the single copy nuclear gene is actin or β2microglobulin.

20. The method according to any of paragraphs 17 to 20, wherein the region of the mitochondrial genome is a mitochondrial gene.

21. The method according to paragraph 20, wherein the mitochondrial gene is cytochrome B.

22. An assay method for identifying one or more agents that modulate microvascular disease, kidney disease and/or cardiovascular disease in a diabetic subject, comprising the steps of:

(a) providing a sample comprising one or more mitochondria;

(b) contacting said sample with one or more agents; and

(c) measuring the activity of the mitochondria in the sample in the presence and absence of said agent(s),

wherein a difference between a) the mitochondrial activity in the presence of the agent and b) the mitochondrial activity in the absence of the agent is indicative that the agent(s) can modulate the one or more disease(s) in the diabetic subject.

23. An agent obtained or obtainable by the method of paragraph 22.

24. A method for preventing and/or treating microvascular disease, kidney disease and/or cardiovascular disease in a diabetic subject comprising administering to said subject one or more agents according to paragraph 23 and/or one or more agents that modulate mitochondrial activity.

25. Use of agent according to paragraph 23 and/or agent that modulates mitochondrial activity in the manufacture of a medicament for the prevention and/or treatment of microvascular disease, kidney disease and/or cardiovascular disease.

26. The method according to paragraph 24 or the use according to paragraph 25, wherein said agent(s) modulate mitochondrial activity in a renal cell.

27. A method for identifying one or more genes that are involved in the presentation of microvascular disease, kidney disease and/or cardiovascular disease, comprising the step of:

(a) providing a test sample from a subject suffering from microvascular disease, kidney disease and/or cardiovascular disease; and

(b) determining the expression profile of one or more mitochondrial genes in said test sample,

wherein a difference in the expression profile of one or more mitochondrial genes in said test sample as compared to a sample from a control subject is indicative that said gene(s) are involved in the presentation of microvascular disease, kidney disease and/or cardiovascular disease in a diabetic subject.

28. A gene(s) obtained or obtainable by the method according to paragraph 27.

**Table 1**

| Differentially expressed clones isolated from the kidneys of the GK rat. | |
|---|---|
| **Clone** | **Identity** |
| CDK1 | Lactate dehydrogenase |
| CDK2 | NADH ubiquinone oxidoreductase 42KD subunit precursor |
| CDK3 | Enach, alpha subunit |
| CDK4 | Beta-defensin* |
| CDK5 | NADH ubiquinone oxidoreductase 42KD subunit precursor |
| CDK6 | Megalin |
| CDK7 | MG87 (NOVEL GENE) |
| CDK8 | ubiquitin-like gene 8 BC084728 |
| CDK9 | Phophatase inhibitor |
| CDK10 | Ubiquitin like novel |
| CDK101 | Hepatoma derived growth factor* (not conclusive) |

(continued)

| Differentially expressed clones isolated from the kidneys of the GK rat. | |
|---|---|
| **Clone** | **Identity** |
| CDK102 | H-protein |
| CDK103 | 16S rRNA |
| CDK104 | Chemokine CXC16 |
| CDK105 | Novel, NLS * |
| CDK106 | Zfn1, novel zinc finger protein |
| CDK107 | Schlafen 4 |
| CDK108 | Mu-crystallin * |
| CDK109 | RM 16S rRNA |
| CDK110 | mitochondrial cytochrome B |
| CDK111 | Glutothione peroxidase |
| CDK112 | Glutaredoxin?? |
| CDK113 | MHC-assoc. invariant chain |
| CDK114 | thiazide sensisitve sodium channel rTSC1 isoform |
| CDK115 | pyruvate dehydrogenase E1 alpha (reduced in GK kidneys |
| CDK116 | RM NADH dehrogenase subunit |
| CDK117 | RM cytochrome C oxidase subunit I |
| CDK118 | RM cytochrome C oxidase subunit I |
| CDK119 | RM 16S rRNA |
| CDK120 | RM cytochrome C oxidase subunit II |
| CDK121 | RM NADH dehrogenase subunit 5 |
| CDK122 | RM 16S rRNA |
| CDK123 | RM cytochrome C oxidase subunit I |
| CDK124 | RM cytochrome C oxidase subunit I |
| CDK125 | RM cytochrome C oxidase subunit I |
| CDK126 | RM cytochrome C oxidase subunit I |

Isolation of candidate diabetes Associated kidney genes (CDKs) on the basis of altered renal mRNA levels during the progression of diabetes in the GK rat. CDK1 to 10 were isolated using differential display (Page et al., 1997) and CDK101 to 126 were isolated using differential screening (Malik et al.,). All these clones showed increased mRNA expression during progressive hyperglycemia in the kidneys of the GK rat at the ages of 6 weeks and 26 weeks. DNA sequencing was used to determine the identity of each clone.

**Table 2**

| Group | Mean | 95% Confidence Interval | Standard Error |
|---|---|---|---|
| Healthy controls | 653 | 492-814 | 333 |
| Diabetics without nephropathy | 577 | 217-937 | 144 |
| Diabetic nephropaths | 2178 | 1556-2798 | 1289 |

SEQUENCES

**Rat cytochrome B** (Accession number AC_000022) PCR product size: 370 bp, Annealing temperature: 60°C
Forward: ctgccgagacgtaaac
Reverse: ttatcgccgcccttgc
**Rat B2 microglobulin** (Accession number NM_012512) PCR product size: 211 bp, Annealing temperature: 60°C
Forward: tgccatt cagaaaactc cc
Reverse: acatcc tggctcacac tgaa
**Rat Actin (rActin)** (Accession number: NM_031144) PCR product size: 299bp, Annealing temperature: 55°C
Forward: acggtcagg tcatcactat c
Reverse: tctgtgtg gattggtggc t
**Human Cytochrome B (hCYTB)** (Accession number: NC_001807) PCR product size: 157bp, Annealing temperature: 60°C
Forward: cctcatcctagcaataatccccatcctccatatat
Reverse: acccttttac catcattgga caagt
**Human Beta 2 micoroglobulin (hB2M)** (Accession number: NM_00404) PCR product size: 86 bp , Annealing temperature: 60°C
Forward: ctgtctccatgtttgatgtatct
Reverse: caacttagaggtggggagcagaga

REFERENCES

1. Ritz, E., Rychlik, I., Locatelli, F. and Halimi, S..End stage renal failure in type 2 diabetes: a medical catastrophe of worldwide dimensions. (1999) Am J of Kid Dis. 34, 795-808.

2. Groop, P., Forsblom, C. and Thomas, M.C. (2005) Mechanisms of Disease: pathway-selective insulin resistance and micorvascular complications of diabetes. Nat. Clin. Pract. Endocrin. and Metab. 1, 100-110.

3. Wild, S., Roglic, G., Green, A., Sicree, R. and King, H. (2004) Global prevalence of diabetes: estimates for the year 2000 and projections for 2030. Diabetes Care. 27, 1047-1053.

4. Stehouwer C.D.A & Smulders Y.M. (2006) Microalbuminuria and risk for cardiovascular disease: analysis of potential mechanisms". J.Am.Soc.Nephrol. 17, 2106-2111.

5. Brownlee, M. (2005) The pathobiology of diabetic complications. Diabetes. 54, 1615-1625.

6. Mason, R., and Wahab, N.A. (2003) Extracellular matrix metabolism in diabetic nephropathy: frontiers in nephrology. J Am. Soc. Nephrol. 14, 1358-1373.

7. Lee, H.B., Yu, M.-R., Yang, Y., Jiang,Z. and Ha, H. (2003) Reactive Oxygen Species-Regulated Signaling Pathways in Diabetic Nephropathy. J. Am. Soc. Nephrol. 14, S241-S245

8. Inoguchi, T., Li, P., Umeda, F., Yu, H.Y., Kakimoto, M., Imamura, M., Aoki, T., Etoh, T., Hashimoto, T., Naruse, M., Sano, H., Utsumi, H. and Nawata, H. (2000) High glucose level and free fatty acid stimulate reactive oxygen species production through protein kinase C dependent activation of NAD(P)H oxidase in cultured vascular cells. Diabetes. 49, 1939-1945

9. Lee, B.H., Ha, H. and King, G.L. (2003) Reactive Oxygen species and diabetic nephropathy. J. Am. Soc. Nephr. 14, S209-S210

10. Kumar Das, S. and Elbein, S.C. (2006) The Genetic Basis of Type 2 Diabetes. Cell Science. 2,100-131

11. Nosadini, R. and Tonolo, G. (2004) Relationship between blood glucose control, pathogenesis and progression of diabetic nephropathy. J. Am. Soc. Nephrol. 15, S1-S5.

12. Sheetz, M.J. and King GL. (2002) Molecular understanding of hyperglycemia's adverse effects for diabetic complications. J. Am. Med. Assoc. 288, 2579D-2588D

13. Wada, J., Makino, H. and Kanwar, Y.S. (2002) Gene Expression and identification of gene therapy targets in diabetic nephropathy. Kid. Int., 61, Symposium 1, S73-S78.

14. Page,R.A., Morris, C.A., Williams, J.D., von Ruhland,C.J., and Malik, A.N. (1997) Isolation of diabetes-associated kidney genes using differential display. Biochem Biophys Res Comm. 232, 49-53.

15. Goto, Y, and Kakizaki, M. (1981) The spontaneous diabetes rat: a model of non-insulin-dependent diabetes mellitus. Pro. Japan Acad. 57, 381-384,

16. Goto, Y., Kakizaki, M., and Masaki, N. (1975) Spontaneous diabetes produced by selective breeding of normal Wistar rats. Pro. Japan Acad. 51, 80-85.

17. Phillips, A.O., Baboolal, K., Riley, S., Grone, H., Janssen, U., Steadman, R., Williams, J., and Floege, J. (2001) Association of prolonged hyperglycemia with glomerular hypertrophy and renal basement membrane thickening in the Goto Kakizaki model of non-insulin-dependent mellitus. Am. J. Kid. Dis. 37, 400-410.

18. Schrijvers, B.F., De Vriese, A.S., Van de Voorde, J., Rasch, R., Lameire, N.H., and Flyvbjerg, A. (2004). Long term renal changes in the GK rat, a model of lean type 2 diabetes. Nephro. Dia. Transplant. 19, 1092-1097.

19. Page, R.A., and Malik, A.N. (2003) Elevated levels of beta defensin-1 mRNA in diabetic kidneys of GK rats. Biochem. Biophys. Res. Commun. 310, 513-21.

20. Malik,A.N., and Al-Kafaji, G. (2007) . Glucose regulation of β-defensin-1 mRNA in human renal cells Biochemical and Biophysical Research Communications 353:318-323

21. Malik, A.N., Zaidi, Q., Morris, C.A. and Williams, J.D. (1997) Cloning of abundantly expressed candidate diabetes associated kidney genes. J. Am. Soc. Nephrol. 8, A2995-A2995.

22. Haneda, M., Koya, D.and Kikkawa, R. (2001) Cellular mechanisms in the development and progression of diabetic nephropathy: activation of the DAG-PKC-ERK pathway. Am. J. Kid. Dis. 38, S178-S181.

23. Goruppi, S., Bonventre, J.V. and Kyriakis, J.M. (2002) Signaling pathways and late-onset gene induction associated with renal mesangial cell hypertrophy. EMBO J. 21, 5427-5436

24. Reeves, W.B. and Andreoli, T.E. (2000) Transforming Growth Factor β contributes to progressive diabetic nephropathy. 97, 7667-7669

25. Brownlee, M., Cerami, A. and Vlassara, H. (1998) Advanced Glycosylation End Products in tissue and the biochemical basis of diabetic complications. N. Engl. J. Med. 318, 1315-1321

26. Way, K.J., Katai, N. and King, G.L. (2001) Protein kinase C and the development of diabetic vascular complications. Diabet. Med.18,945-959.

All publications mentioned in the above specification are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in biochemistry and molecular biology or related fields are intended to be within the scope of the following claims.

**Claims**

1. A method for identifying a subject having or at risk of developing one or more diseases resulting from oxidative stress comprising the steps of:

   (a) providing a test sample from said subject; and
   (b) measuring at least one indicator of altered mitochondrial activity in the test sample,
   wherein a difference in mitochondrial activity in the test sample as compared to a control is indicative that said subject has or is at risk of developing said disease(s); and
   wherein the at least one indicator of altered mitochondrial activity is an alteration in the mitochondrial DNA content or mitochondrial copy number in the test sample as compared to the control.

2. A method according to any preceding claim, wherein an alteration in mitochondrial DNA content is indicative of disease.

3. A method according to claim 1 or claim 2, wherein the disease is selected from the group consisting of Alzheimers disease, microvascular disease, kidney disease, cardiovascular disease, schizophrenia, Parkinsons disease, Huntingdon's chorea, metal-induced toxicity in diabetes, cancer, anaemia, hypertension, atherosclerosis, diabetes, muscular dystrophy and aging or a combination of at least two of said diseases.

4. A method according to claim 3, wherein the disease is Alzheimers disease.

5. A method according to any of the preceding claims, wherein the test sample is or is derived from a blood sample, a biopsy specimen, a tissue explant, an organ culture or a cultured cell.

6. The method according to any of the preceding claims, wherein the test sample is nucleic acid that is or is derived from a blood sample, a biopsy specimen, a tissue explant, an organ culture or a cultured cell.

7. The method according to claim 6, wherein the nucleic acid is genomic DNA and/or total RNA.

8. A method according to claim 6, wherein the cultured cell is a cultured renal cell.

9. The method according to claim 6 or 8, wherein said cultured cell is grown in high or low glucose prior to measuring the at least one indicator of mitochondrial activity.

10. An assay method for identifying one or more agents that modulate one or more diseases resulting from oxidative stress in a subject, comprising the steps of:

    (a) providing a sample comprising one or more mitochondria;
    (b) contacting said sample with one or more agents; and
    (c) measuring the activity of the mitochondria in the sample in the presence and absence of said agent(s), wherein a difference between a) the mitochondrial activity in the presence of the agent and b) the mitochondrial activity in the absence of the agent is indicative that the agent(s) can modulate the one or more disease(s) in the subject, wherein the at least one indicator of altered mitochondrial activity is an alteration in the mitochondrial DNA content or mitochondrial copy number in the sample in the presence and absence of said agent(s).

Figure 1

Mitochondrial copy numbers in normal (c) and diabetic (GK) rat kidneys

Figure 2

Effect of high glucose on mitochondrial copy
numbers in human mesangial cells

## Figure 3

(a)

mt copy number in healthy controls, diabetics without nephropathy and diabetics with nephropathy

(b)

Mt copy numbers in DN patients and controls (averages with standard deviation)

(c)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6852541 B
- US 6541267 B
- US 6096556 A
- EP 0774464 A
- US 5798035 A
- US 5789172 A
- US 5751629 A


**Non-patent literature cited in the description**

- **WILD S et al.** *Diabetes Care,* 2004, vol. 27, 1047-1053
- **GERBITZ, K. D. et al.** *Biochim. Biophys. Acta,* 1995, vol. 1271, 253-260
- **SHOFFNER, J. M. ; WALLACE, D. C.** Oxidative phosphorylation diseases. *The Metabolic and Molecular Bases of Inherited Disease,* vol. 1, 1535-1610
- **SHIN, C. S.** *J. Kor. Diabetes Asso.,* 1994, vol. 18, 344-350
- **ROLO ; PALMEIRA.** *Toxicology and Applied Pharmacology,* 2006, vol. 212, 167-178
- **FLORA.** Role of free radicals in antioxidants in health and disease. *Cell Mol. Biol,* 2007, vol. 53-1, 1-2
- **GOHIL et al.** *L Appl. Physiol.,* 1988, vol. 64, 115-119
- **DUTHIE et al.** *Arch. Biochem. Biophys.,* 1990, vol. 282, 78-83
- **SASTRE et al.** *Am. J. Physiol.,* 1992, vol. 263, R992-R995
- **SEN et al.** *J. Appl. Physiol.,* 1994, vol. 76, 2570-2577
- **HACK et al.** *BLOOD,* 1998, vol. 92, 59-67
- **FENDRI et al.** Oxidative stress: involvement in schizophrenia pathophysiology:a review. *Encephale,* March 2006, vol. 32, 244-52
- **VALKO et al.** Metals, toxicity and oxidative stress. *Current Medicinal Chemistry,* 2005, vol. 12 (10), 1161-1208
- **VALKO et al.** Free radicals, metals and antioxidants in oxidative stress-induced cancer. *Chemico-biological Interactions,* 2006, vol. 160, 1-40
- **WILLCOX et al.** Antioxidants and prevention of chronic disease. *Crtic Rev. Food Sci. Nutr.,* 2004, vol. 44, 275-295
- **B.VAN HOUTEN et al.** Role of mitochondrial DNA in toxic responses to oxidative Stress. *DNA Repair (Amst,* 2006, vol. 5, 145-152
- **TIDBALL ; WEHLIN-HENRICKS.** The role of free radicals in the pathophysiology of muscular dystrophy. *J Appl Physiol.,* 2007, vol. 102, 1677-1686
- **STUVELING et al.** *Nephrol.Dial.Transpl.,* 2005, vol. 20, 497-508
- **MEHRABIAN et al.** *J. Neurochemistry,* 2005, vol. 93, 850-860
- **CRUZ-ORIVE et al.** *Am. J. Physiol.,* 1990, vol. 258, L148
- **SCHWERZMANN et al.** *J. Cell Biol.,* 1986, vol. 102, 97
- **MILLER et al.** *J. Neurochem.,* 1996, vol. 67, 1897
- **FAHY et al.** *Nucl. Ac. Res.,* 1997, vol. 25, 3102
- **LEE et al.** *Diabetes Res. Clin. Practice,* 1998, vol. 42, 161
- **LEE et al.** *Diabetes,* 1997, vol. 46 (1), 175A
- **WILLIAMS.** *J. Biol.Chem,* 1986, vol. 261 (26), 12390-12394
- **SPRINGER et al.** *Brain Research Protocols,* 1998, vol. 2, 259-263
- **ABU-AMERO ; BOSLEY.** *Arch Pathol.Lab Methods,* 2005, vol. 129, 1295-1298
- **ARTUCH et al.** *Clinical Biochemistry,* 2000, vol. 33 (6), 481-485
- Current Protocols in Immunology. John Wiley & Sons, 1998
- **GAVIN et al.** Diabetes Care. American Diabetes Association Expert Committee on the Diagnosis and Classification of Diabetes Mellitus, 1999, vol. 22, S5-S19
- *Genomics,* 1989, vol. 4, 560
- *Science,* 1988, vol. 241, 1077
- *Proc. Nat. Acad. Sci. USA,* 1992, vol. 89, 392-396
- *Bio/Technology,* 1988, vol. 6, 1197-1202
- *Bio/Technology,* 1995, vol. 13, 563-565
- *Proc. Nat. Acad. Sci. USA,* 1990, vol. 87, 1874-1878
- *Science,* 1988, vol. 239, 487

- **FAHY et al.** *Nucl. Acids Res.,* 1997, vol. 25, 3102
- **GHOSH et al.** *Am. J. Hum. Genet.,* 1996, vol. 58, 325
- **BARANY.** *Proc. Nat. Acad. Sci.,* 1991, vol. 88, 189
- **CAHILL et al.** *Clin. Chem.,* 1991, vol. 37, 1482
- **LIZARDI et al.** *Biotechnol.,* 1988, vol. 6, 1197
- **FOX et al.** *J. Clin. Lab. Analysis,* 1989, vol. 3, 378
- **CLONEY et al.** *Clin. Chem.,* 1994, vol. 40, 656
- **BAGOCKA et al.** *Diabetes,* 2005, vol. 54, 1392-1399
- **KOMMINOTH.** *Diagn. Mol. Pathol.,* 1992, vol. 1, 142-150
- **KOMMINOTH et al.** *Histochemistry,* 1992, vol. 98, 217-228
- **SAMBROOK et al.** Molecular Cloning, A Laboratory Manual. 1989
- **DE VRIESE et al.** *J. Am Soc Nephrol,* 2001, vol. 12, 993-1000
- **FLYVBJERG et al.** *Diabetes,* 2002, vol. 51, 3090-3094
- **ZIYADEH et al.** *Proc Natl Acad Sci,* 2000, vol. 97, 8015-8020
- **CHEN et al.** *Biochem Biophys Res Commun,* 2003, vol. 300, 16-22
- **BENIGNI et al.** *J. Am Soc Nephrol,* 2003, vol. 14, 1816-1824
- *Int. J. Urol & Nephrol.,* 2005, vol. 37, 655-663
- **J. SAMBROOK ; E. F. FRITSCH ; T. MANIATIS.** Molecular Cloning: A Laboratory Manual. Cold Spring Harbor Laboratory Press, 1989
- **AUSUBEL, F. M. et al.** Current Protocols in Molecular Biology. John Wiley & Sons, 1995
- **B. ROE ; J. CRABTREE ; A. KAHN.** DNA Isolation and Sequencing: Essential Techniques. John Wiley & Sons, 1996
- **J. M. POLAK ; JAMES O'D. MCGEE.** Situ Hybridization: Principles and Practice. University Press, 1990
- Oligonucleotide Synthesis: A Practical Approach. Irl Press, 1984
- Methods of Enzymology: DNA Structure Part A: Synthesis and Physical Analysis of DNA. **D. M. J. LILLEY ; J. E. DAHLBERG.** Methods in Enzymology. Academic Press, 1992
- **RITZ, E. ; RYCHLIK, I. ; LOCATELLI, F. ; HALIMI, S.** End stage renal failure in type 2 diabetes: a medical catastrophe of worldwide dimensions. *Am J of Kid Dis.,* 1999, vol. 34, 795-808
- **GROOP, P. ; FORSBLOM, C. ; THOMAS, M.C.** Mechanisms of Disease: pathway-selective insulin resistance and micorvascular complications of diabetes. *Nat. Clin. Pract. Endocrin. and Metab.,* 2005, vol. 1, 100-110

- **WILD, S. ; ROGLIC, G. ; GREEN, A. ; SICREE, R. ; KING, H.** Global prevalence of diabetes: estimates for the year 2000 and projections for 2030. *Diabetes Care,* 2004, vol. 27, 1047-1053
- **STEHOUWER C.D.A ; SMULDERS Y.M.** Microalbuminuria and risk for cardiovascular disease: analysis of potential mechanisms. *J.Am.Soc.Nephrol.,* 2006, vol. 17, 2106-2111
- **BROWNLEE, M.** The pathobiology of diabetic complications. *Diabetes,* 2005, vol. 54, 1615-1625
- **MASON, R. ; WAHAB, N.A.** Extracellular matrix metabolism in diabetic nephropathy: frontiers in nephrology. *J Am. Soc. Nephrol.,* 2003, vol. 14, 1358-1373
- **LEE, H.B. ; YU, M.-R. ; YANG, Y. ; JIANG,Z. ; HA, H.** Reactive Oxygen Species-Regulated Signaling Pathways in Diabetic Nephropathy. *J. Am. Soc. Nephrol.,* 2003, vol. 14, S241-S245
- **INOGUCHI, T. ; LI, P. ; UMEDA, F. ; YU, H.Y. ; KAKIMOTO, M. ; IMAMURA, M. ; AOKI, T. ; ETOH, T. ; HASHIMOTO, T. ; NARUSE, M.** High glucose level and free fatty acid stimulate reactive oxygen species production through protein kinase C dependent activation of NAD(P)H oxidase in cultured vascular cells. *Diabetes,* 2000, vol. 49, 1939-1945
- **LEE, B.H. ; HA, H. ; KING, G.L.** Reactive Oxygen species and diabetic nephropathy. *J. Am. Soc. Nephr.,* 2003, vol. 14, S209-S210
- **KUMAR DAS, S. ; ELBEIN, S.C.** The Genetic Basis of Type 2 Diabetes. *Cell Science,* 2006, vol. 2, 100-131
- **NOSADINI, R. ; TONOLO, G.** Relationship between blood glucose control, pathogenesis and progression of diabetic nephropathy. *J. Am. Soc. Nephrol.,* 2004, vol. 15, S1-S5
- **SHEETZ, M.J. ; KING GL.** Molecular understanding of hyperglycemia's adverse effects for diabetic complications. *J. Am. Med. Assoc.,* 2002, vol. 288, 2579D-2588D
- **WADA, J. ; MAKINO, H. ; KANWAR, Y.S.** Gene Expression and identification of gene therapy targets in diabetic nephropathy. *Kid. Int.,* 2002, vol. 61 (1), S73-S78
- **PAGE,R.A. ; MORRIS, C.A. ; WILLIAMS, J.D. ; VON RUHLAND,C.J. ; MALIK, A.N.** Isolation of diabetes-associated kidney genes using differential display. *Biochem Biophys Res Comm.,* 1997, vol. 232, 49-53
- **GOTO, Y ; KAKIZAKI, M.** The spontaneous diabetes rat: a model of non-insulin-dependent diabetes mellitus. *Pro. Japan Acad.,* 1981, vol. 57, 381-384
- **GOTO, Y. ; KAKIZAKI, M. ; MASAKI, N.** Spontaneous diabetes produced by selective breeding of normal Wistar rats. *Pro. Japan Acad.,* 1975, vol. 51, 80-85

- **PHILLIPS, A.O. ; BABOOLAL, K. ; RILEY, S. ; GRONE, H. ; JANSSEN, U. ; STEADMAN, R. ; WILLIAMS, J. ; FLOEGE, J.** Association of prolonged hyperglycemia with glomerular hypertrophy and renal basement membrane thickening in the Goto Kakizaki model of non-insulin-dependent mellitus. *Am. J. Kid. Dis.,* 2001, vol. 37, 400-410
- **SCHRIJVERS, B.F. ; DE VRIESE, A.S. ; VAN DE VOORDE, J. ; RASCH, R. ; LAMEIRE, N.H. ; FLYVBJERG, A.** Long term renal changes in the GK rat, a model of lean type 2 diabetes. *Nephro. Dia. Transplant.,* 2004, vol. 19, 1092-1097
- **PAGE, R.A. ; MALIK, A.N.** Elevated levels of beta defensin-1 mRNA in diabetic kidneys of GK rats. *Biochem. Biophys. Res. Commun.,* 2003, vol. 310, 513-21
- **MALIK,A.N. ; AL-KAFAJI, G.** Glucose regulation of β-defensin-1 mRNA in human renal cells. *Biochemical and Biophysical Research Communications,* 2007, vol. 353, 318-323

- **MALIK, A.N. ; ZAIDI, Q. ; MORRIS, C.A. ; WILLIAMS, J.D.** Cloning of abundantly expressed candidate diabetes associated kidney genes. *J. Am. Soc. Nephrol.,* 1997, vol. 8, A2995-A2995
- **HANEDA, M. ; KOYA, D. ; KIKKAWA, R.** Cellular mechanisms in the development and progression of diabetic nephropathy: activation of the DAG-PKC-ERK pathway. *Am. J. Kid. Dis.,* 2001, vol. 38, S178-S181
- **GORUPPI, S. ; BONVENTRE, J.V. ; KYRIAKIS, J.M.** Signaling pathways and late-onset gene induction associated with renal mesangial cell hypertrophy. *EMBO J.,* 2002, vol. 21, 5427-5436
- **REEVES, W.B. ; ANDREOLI, T.E.** *Transforming Growth Factor β contributes to progressive diabetic nephropathy,* 2000, vol. 97, 7667-7669
- **BROWNLEE, M. ; CERAMI, A. ; VLASSARA, H.** Advanced Glycosylation End Products in tissue and the biochemical basis of diabetic complications. *N. Engl. J. Med.,* 1998, vol. 318, 1315-1321
- **WAY, K.J. ; KATAI, N. ; KING, G.L.** Protein kinase C and the development of diabetic vascular complications. *Diabet. Med.,* 2001, vol. 18, 945-959